# EUROPEAN PATENT SPECIFICATION

(11) **EP 2 818 170 B1**
(45) Date of publication and mention of the grant of the patent: **05.12.2018**
(21) Application number: 14186203.7
(22) Date of filing: 09.09.2010
(51) Int. Cl.: A61K 31/502, A61P 35/00, A61P 35/02

(54) **N-(4-((3-(2-amino-4-pyrimidinyl)-2-pyridin yl)oxy)phenyl)-4-(4-methyl-2-thienyl)-1-phthalazinamine for use in the treatment of antimitotic agent resistant cancer**
N-(4-((3-(2-Amino-4-pyrimidinyl)-2-pyridin yl)oxy)phenyl)-4-(4-methyl-2-thienyl)-1-phthalazinamin zur Behandlung von Krebs mit Resistenz gegenüber Antimitotika
N-(4-((3-(2-amino-4-pyrimidinyl)-2-pyridine yl) oxy)phényl)-4-(4-méthyl-2-thiényl)-1-phthalazinamine à utiliser dans le traitement d'un cancer résistant aux agents antimitotiques

(30) Priority: 11.09.2009 US 241527 P
(43) Date of publication of application: 31.12.2014
(62) Divisional of application: 10755044.4
(73) Proprietor: Amgen, Inc, Thousand Oaks, CA 91320-1799 (US)
(72) Inventor: Payton, Marc, Newbury Park, CA California 91320 (US); Kendall, Richard, Thousand Oaks, CA California 91360 (US)
(74) Representative: Grünecker Patent- und Rechtsanwälte PartG mbB

(56) References cited:
- US-A1- 2007 185 111
- Harrington E A: "VX-680, a potent and selective small-molecule inhibitor of the Aurora kinases, suppresses tumor growth in vivo", Nature medicine , vol. 10, no. 3 March 2004 (2004-03), pages 262-267, XP002612560, Retrieved from the Internet: URL:http://www.nature.com/nm/journal/v10/n 3/pdf/nm1003.pdf [retrieved on 2010-12-02]
- MOUNTZIOS ET AL: "Aurora kinases as targets for cancer therapy", CANCER TREATMENT REVIEWS, SAUNDERS, US, vol. 34, no. 2, 19 November 2007 (2007-11-19), pages 175-182, XP022532141, ISSN: 0305-7372
- ANAND S ET AL: "AURORA-A AMPLIFICATION OVERRIDES THE MITOTIC SPINDLE ASSEMBLY CHECKPOINT, INDUCING RESISTANCE TO TAXOL", CANCER CELL, CELL PRESS, US, vol. 3, no. 1, 1 January 2003 (2003-01-01) , pages 51-62, XP009026392, ISSN: 1535-6108, DOI: DOI:10.1016/S1535-6108(02)00235-0
- BUSH TAMMY L ET AL: "Preclinical characterization of AMG 900, an orally available small molecule inhibitor of aurora kinases in phase 1 clinical trials", PROCEEDINGS OF THE ANNUAL MEETING OF THE AMERICAN ASSOCIATION FOR CANCER RESEARCH, AMERICAN ASSOCIATION FOR CANCER RESEARCH, US, vol. 51, 1 April 2010 (2010-04-01), page 1076, XP009142030, ISSN: 0197-016X
- COUMAR M S ET AL: "Avances in Aurora kinase inhibitor patents", EXPERT OPINION ON THERAPEUTIC PATENTS,, vol. 19, no. 3, 1 March 2009 (2009-03-01), pages 321-356, XP002543182, ISSN: 1354-3776, DOI: 10.1517/13543770802646949

## Description

### RELATED APPICATIONS

This application claims the benefit of U.S. Provisional Application No. 61/241,527, filed 11 September 2009.

### FIELD OF THE INVENTION

The present invention relates to the use of N-(4-((3-(2-amino-4-pyrimidinyl) -2-pyridinyl)oxy)phenyl)-4-(4-methyl-2-thienyl)-1-phthalazinamine for treating cancers, including solid tumors, which have become resistant to treatment with antimitotic agents and/or other chemotherapeutic agents.

### BACKGROUND OF THE INVENTION

Cancer is one of the most widespread diseases affecting Mankind, and a leading cause of death worldwide. In the United States alone, cancer is the second leading cause of death, surpassed only by heart disease. Cancer is often characterized by deregulation of normal cellular processes or unregulated cell proliferation. Cells that have been transformed to cancerous cells tend to proliferate in an uncontrolled and unregulated manner leading to, in some cases, metastisis or the spread of the cancer. Deregulation of the cell proliferation could result from the modification to one or more genes, responsible for the cellular pathways that control cell-cycle progression. Or it could result from DNA modifications (including but not limited to mutations, amplifications, rearrangements, deletions, and epigenetic gene silencing) in one or more cell-cycle checkpoint regulators which allow the cell to move from one phase of the cell cycle to another unchecked. Another way is that modifications in cellular machinery itself could result in mitotic errors that are not properly detected or repaired, and the cell could be allowed to move through the cell cycle unchecked.

Mitosis is the process by which a eukaryotic cell segregates its duplicated chromosomes into two identical daughter nuclei. It is generally followed immediately by cytokinesis, which divides the nuclei, cytoplasm, organelles and cell membranes into two daughter cells containing roughly equal shares of these cellular components. Mitosis and cytokinesis together define the mitotic (M) phase of the cell cycle - the division of the mother cell into two daughter cells, genetically identical to each other and to their parent cell.

The process of mitosis is complex and highly regulated. The sequence of events is divided into distinct phases, corresponding to the completion of one set of activities and the start of the next. These stages are prophase, prometaphase, metaphase, anaphase and telophase. During the process of mitosis duplicated chromosomes condense and attach to fibers that pull the sister chromatids to opposite sides of the cell. The cell then divides in cytokinesis, to produce two identical daughter cells. Errors in mitosis can either kill a cell through apoptosis or cause mis-segratation of chromosomes that may lead to cancer.

Normally, cell-cycle checkpoints are activated if DNA errors are detected (e.g. DNA damage). If these errors to the genome cannot be fixed, the cell normally undergoes apoptosis. However, if the cell is allowed to move through its cell-cycle and progress unchecked, then more mutations can accumulate over time. These gene modifications can accrue and eventually leading cell progeny with pre-malignant or malignant neoplastic characteristics (e.g. uncontrolled proliferation) through adaptation.

Antimitotic agents are anti-cancer agents that inhibit the function of microtubules. Microtubules are protein polymers formed by α-tubulin and β-tubulin heterodimers that play an important role in the formation of the mitotic spindle apparatus and cytokinesis at the end of mitosis. Anti-cancer agents that target microtubules represent a proven approach for intervening in the proliferation of cancer cells.

Several classes of antimitotic agents have been developed as anticancer agents. Taxanes are the most prominent class of antimitotic agent that includes paclitaxel (taxol) and docetaxel (taxotere). The vinca alkaloids are a class of microtubule-destabilzing agents that includes vincristine, vinblastine, vindesine, and vinorelbine. Other emerging class includes the epothilones (ixabepilone). These antimitotic agents act to prevent the proliferation of cancer cells by either stabilizing- or destabilizing-microtubules. This direct inhibition of microtubules results in cell arrest and death through apoptosis or mitotic catastrophe. Paclitaxel was the first compound of the taxane series to be discovered. Docetaxel, a structural analog of paclitaxel, was later discovered. Paclitaxel and docetaxel are commonly used to treat a variety of human malignancies, including ovarian cancer, breast cancer, head and neck cancer, lung cancer, gastric cancer, esophageal cancer, prostate cancer, and AIDS-related Kaposi's sarcoma. The primary side effect of taxanes is myelosupression, primarily neutropenia, while other side effects include peripheral edema, and neurotoxicity (peripheral neuropathy).

Resistance to taxanes is a complicating factor to successful cancer treatment and is often associated with increased expression of the *mdr-1* encoded gene and its product, the P-glycoprotein (P-gp). Other documented mechanisms of acquired resistance to taxanes include tubulin mutations, overexpression, amplification, and isotype switching). Mutations in α- or β-tubulin inhibit the binding of taxanes to the correct place on the microtubules; this renders the drug ineffective. In addition, some resistant cells also display increased aurora kinase, an enzyme that promotes completion of mitosis.

The vinca alkaloids (*Vincas*; also referred to as plant alkaloids), are able to bind to the β-tubulin subunit of microtubules, blocking their ability to polymerize with the α-tubulin subunit to form complete microtubules. This causes the cell cycle to arrest in metaphase leading to apoptotic cell death because, in absence of an intact mitotic spindle, duplicated chromosomes cannot align along the division plate. Research has identified dimeric asymmetric vinca alkaloids: vinblastine, vincristine, vinorelbine, and vindesine, each of which is useful in the treatment of cancer, including bladder and testicular cancers, Kaposi's sarcoma, neuroblastoma and Hodgkin's disease, and lung carcinoma and breast cancer. The major side effects of vinca alkaloids are that they can cause neurotoxicity and myleosupression in patients.

Resistance to the vinca alkaloids can occur rapidly in experimental models. Antitumor effects of vinca alkaloids can be blocked in multidrug resistant cell lines that overexpress ATP-binding cassette (ABC) transporter-mediated drug efflux transporters such as P-gp and MRP1. Other forms of resistance stem from mutations in β-tubulin that prevent the binding of the inhibitors to their target.

Other chemotherapeutic agents include topoisomerase inhibitors, such as irinotecan and topotecan (type I inhibitors) and amsacrine, etoposide, etoposide phosphate and tenoposide (type II inhibitors). Topoisomerase inhibitors affect DNA synthesis and, in particular, work by preventing transcription and replication of DNA.

Yet another class of chemotherapeutic agents is the anthracycline antibiotics class including daunorubicin, doxorubicin, idarubicin, epirubicin, and mitoxantrone. Today, anthracyclines are used to treat a large number of cancers including lymphomas, leukemias, and uterine, ovarian, lung and breast cancers. Anthracyclines work by forming free oxygen radicals that breaks DNA strands thereby inhibiting DNA synthesis and function. One of the main side effects of anthracyclines is that they can damage cells of heart muscle leading to cardiac toxicity.

Resistance to anticancer agents, including, without limitation, chemotherapeutic agents and antimitotic agents, has become a major drawback in the treatment of cancer. Such resistance has resulted in patients becoming cross-resistant to the effects of many different drugs. More particularly, multidrug resistence is a problem. Further, such resistance to anticancer treatment(s) inevitably leads to patient death. Consequently, development of drug resistance remains a problem with all anticancer therapies and, accordingly, there remains a need to identify a treatment for cancers which are no longer responsive, or are only marginally effective, to cancer treatments, including traditional treatment with chemotherapeutic agents, such as taxanes and vinca alkaloids, as well as anticancer agents undergoing clinical testing for regulatory approval.

### BRIEF DESCRIPTION OF THE DRAWINGS/FIGURES

Figure 1 is a graph depicting the effects of AMG 900 and Taxol on MES-SA and MES-SA Dx5 Cell Lines, p-Histone H3 EC₅₀ Values;
Figure 2 is a graph depicting the effects of AMG 900 and Taxol on NCI-H460 Parent and NCI-H460 Taxol-resistant Cell Lines, Cell Cycle DNA Content EC₅₀ Values;
Figure 3 is a graph depicting the effect of AMG 900 and Taxol on MDA-MB-231 and MDA-MB-231 Taxol-Resistant Cell Lines, Cell Cycle DNA Content EC₅₀ Values;
Figure 4 is a graph illustrating how AMG 900 Inhibits the growth of established MES-SA Dx5 xenograft tumors; and
Figure 5 is a graph depicting the effects of AMG 900 and Taxol Treatment on the Growth of Established NCI-H460-Taxol resistant Xenografts.
Figure 6 is a graph depicting the effects of AMG 900 on HCT116 parental, AZD1152-Resistant HCT116 Cell Lines and Paclitaxel-Resistant Cell Lines.

### BRIEF DESCRIPTION OF THE INVENTION

The present invention provides the following embodiments defined under items 1-9:
1. The compound N-(4-((3-(2-amino-4-pyrimidinyl)-2-pyridinyl)oxy)phenyl)-4-(4-methyl-2-thienyl)-1-phthalazinamine or a pharmaceutically acceptable salt thereof for use in the treatment of cancer in a subject, wherein the cancer is selected from the group consisting of breast cancer, lung cancer, colon cancer, uterine cancer, liver cancer, kidney cancer and multiple myeloma or a combination thereof, and the cancer is refractory to prior treatment with a chemotherapeutic agent, an anti-neoplastic agent, AZD1152, PHA-739538, MK-0457 or a combination thereof.
2. Use of the compound N-(4-((3-(2-amino-4-pyrimidinyl)-2-pyridinyl)oxy)phenyl)-4-(4-methyl-2-thienyl)-1-phthalazinamine or a pharmaceutically acceptable salt thereof for the preparation of a medicament for the treatment of cancer in a subject, wherein the cancer is selected from the group consisting of breast cancer, lung cancer, colon cancer, uterine cancer, liver cancer, kidney cancer and multiple myeloma or a combination thereof, and the cancer is refractory to prior treatment with a chemotherapeutic agent, an anti-neoplastic agent, AZD1152, PHA-739538, MK-0457 or a combination thereof.
3. The use or the compound for use according to item 1 or 2, wherein the chemotherapeutic agent is a taxanes or paclitaxel.
4. The use or the compound for use according to any one of items 1 to 3 wherein the cancer is breast cancer, lung cancer, or multiple myeloma.
5. The use or the compound for use according to item 1 or 2 wherein the cancer is selected from the group consisting of breast cancer, lung cancer, colon cancer, uterine cancer and multiple myeloma or a combination thereof, and the cancer is refractory to prior treatment with an anti-neoplastic agent.
6. The use or the compound for use according to items 1, 2 or 5 wherein the anti-neoplastic agent is a doxorubicin.
7. The use or compound for use according to item 5 wherein the cancer is uterine cancer.
8. The use or compound for use according to item 5 wherein the prior treatment is with AZD1152, or with PHA-739538.
9. The use or compound for use according to any one of items 1 - 8 wherein AMG 900 is administered to the patient in need of treatment in a dosage amount ranging from a daily dose of about 0,1 mg/kg to a daily dose of about 25 mg/kg of the patient.

Disclosed herein is the use of the compound, N-(4-((3-(2-amino-4-pyrimidinyl)-2-pyridinyl)oxy)phenyl)-4-(4-methyl-2-thienyl)-1-phthalazinamine (also referred to herein as "AMG 900" or "the compound") and pharmaceutically acceptable salt forms thereof, for the treatment of advanced cancers, including solid tumors and cancer cells, which are refractory to standard-of-care, government approved antimitotic agents such as taxanes, including paclitaxel and docetaxel and other chemotherapeutic agents, including doxorubicin and other agents being administered in clinical trials for treatment of cancer. AMG 900 has a chemical structure of:

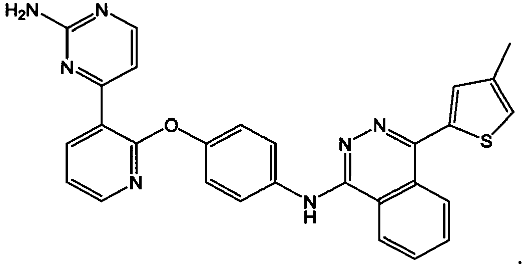

Further disclosed herein is the use of a pharmaceutical composition comprising this compound, or a pharmaceutically acceptable salt form thereof, for therapeutic, prophylactic, acute or chronic treatment of cancer and cancer cells in patients which have been previously treated with chemotherapeutic agents, including anti-mitotic agents. Further disclosed herein is the use of AMG 900 in the manufacture of medicaments and pharmaceutical compositions for methods of treatment of cancer in subjects who have been previously treated with antimitotic agents, including mitotic spindle inhibitors and anti-microtubulin agents, or other drugs used in cancer chemotherapy (also referred to herein as chemotherapeutic agents), including doxorubicin, daunorubicin, dactinomycin, colchicine, vinblastine, vincristine, etoposide and mitoxantrone. Further disclosed herein is a method of treating taxane-resistant tumor types, including non-small cell lung cancer, breast cancer, and hormone refractory prostate cancer in a asubject, the method comprising administering to the subject an effective dosage amount of AMG 900 or a pharmaceutically acceptable salt thereof, to treat the taxane-resistent tumor.

### DETAILED DESCRIPTION OF THE INVENTION

AMG 900, an Aurora kinase inhibitor, has been found to provide a surprising and unexpected advantage over current standard-of-care cancer therapeutic agents that target tubulin (such as paclitaxel, ixabepilone, and vinca alkaloids) and other chemotherapeutic agents (such as doxorubicin), including AZD1152, in human clinical trials. Particularly, AMG 900 delivers efficacy in inhibiting or slowing the progression or growth of tumors that have become cross-resistant to anti-mitotic agents through a variety of proposed mechanisms, including for example, through ATP-binding cassette (ABC) transporter-mediated drug efflux, tubulin gene amplification or modification, or structural alterations in α or β tubulin protein. In addition, AMG 900 targets proliferating cells in the G₂M-phase of the cell cycle and is therefore unlikely to cause the peripheral neuropathy seen with antimitotics that target microtubules.

### DEFINITIONS

The following definitions should further assist in understanding the scope of the invention described herein.

The terms "cancer" and "cancerous" when used herein refer to or describe the physiological condition in subjects that is typically characterized by unregulated cell growth. Examples of cancer include, without limitation, carcinoma, lymphoma, sarcoma, blastoma and leukemia. More particular examples of such cancers include squamous cell carcinoma, lung cancer, pancreatic cancer, cervical cancer, bladder cancer, hepatoma, breast cancer, colon carcinoma, and head and neck cancer. While the term "cancer" as used herein is not limited to any one specific form of the disease, it is believed that the compound for use according to the invention will be particularly effective for cancers, in a subject, which have become resistant in some degree to treatment with anti-cancer agents, including without limitation chemotherapeutic agents, antimitotic agents, anthracyclines and the like, and for cancers which have relapsed post treatment with such anti-cancer agents.

The term "chemotherapeutic agent" when used herein refers to the treatment of a cancer by killing cancerous cells. This term additionally refers to antineoplastic drugs used to treat cancer or a combination of these drugs into a standardized treatment regimen. Examples of chemotherapeutic agents include, without limitation, alkylating agents such as cisplatin, carboplatin, oxaliplatin; alkaloids including vinca alkaloids (examples include vincristine, vinblastine, vinorelbine and vindesine) and taxanes (examples include paclitaxel (Taxol) and docetaxel); topoisomerase inhibitors such as irinotecan, topotecan, amsacrine, etoposide, etoposide phosphate and teniposide; and various antineoplastic agents such as dactinomycin, doxorubicin, epirubicin, bleomycin and others.

The term "comprising" is meant to be open ended, including the indicated component(s) but not excluding other elements.

The term "multidrug resistant" when used herein refers to cancer cells resistant to multiple drugs of different chemical structures and/or resistant to drugs directed at different targets.

The term "refractory" when used here is intended to refer to not-yielding to, resistant or non-responsive to treatment, stimuli (therapy) or cure, including resistance to multiple therapeutic curative agents. "Refractory" when used herein in the context of characterizing a cancer or tumor is intended to refer to the cancer or tumor being non-responsive or having a resistant or diminished response to treatment with one or more anticancer agents. The treatment typically is continual, prolonged and/or repetitive over a period of time resulting in the cancer or tumor developing resistance or becoming refractory to that very same treatment.

The term "subject" as used herein refers to any mammal, including humans and animals, such as cows, horses, dogs and cats. Thus, the invention may be used in human patients as well as in veterinarian subjects and patients. In one embodiment of the invention, the subject is a human.

The phrase "therapeutically-effective" is intended to quantify the amount of the compound (AMG 900), which will achieve a reduction in size or severity of the cancer or tumor over treatment of the cancer by conventional antimitotic cancer therapies, while reducing or avoiding adverse side effects typically associated with the conventional anti-mitotic cancer therapies.

The terms "treat", "treating" and "treatment" as used herein refer to therapy, including without limitation, curative therapy, prophylactic therapy, and preventative therapy. Prophylactic treatment generally constitutes either preventing the onset of disorders altogether or delaying the onset of a pre-clinically evident stage of disorders in individuals.

The term "pharmaceutically-acceptable salts" embraces salts commonly used to form alkali metal salts and to form addition salts of free acids or free bases. The nature of the salt is not critical, provided that it is pharmaceutically-acceptable. Suitable pharmaceutically-acceptable acid addition salts of the compound may be prepared from an inorganic acid or from an organic acid. Examples of such inorganic acids include, without limitation, hydrochloric, hydrobromic, hydroiodic, nitric, carbonic, sulfuric and phosphoric acid. Examples of organic acids include, without limitation, aliphatic, cycloaliphatic, aromatic, arylaliphatic, heterocyclic, carboxylic and sulfonic classes of organic acids, examples of which are formic, acetic, adipic, butyric, propionic, succinic, glycolic, gluconic, lactic, malic, tartaric, citric, ascorbic, glucuronic, maleic, fumaric, pyruvic, aspartic, glutamic, benzoic, anthranilic, mesylic, 4-hydroxybenzoic, phenylacetic, mandelic, embonic (pamoic), methanesulfonic, ethanesulfonic, ethanedisulfonic, benzenesulfonic, pantothenic, 2-hydroxyethanesulfonic, toluenesulfonic, sulfanilic, cyclohexylaminosulfonic, camphoric, camphorsulfonic, digluconic, cyclopentanepropionic, dodecylsulfonic, glucoheptanoic, glycerophosphonic, heptanoic, hexanoic, 2-hydroxy-ethanesulfonic, nicotinic, 2-naphthalenesulfonic, oxalic, palmoic, pectinic, persulfuric, 2-phenylpropionic, picric, pivalic propionic, succinic, tartaric, thiocyanic, mesylic, undecanoic, stearic, algenic, β-hydroxybutyric, salicylic, galactaric and galacturonic acid.

Suitable pharmaceutically-acceptable base addition salts of the compound include, without limitation, metallic salts such as salts made from aluminum, calcium, lithium, magnesium, potassium, sodium and zinc, or salts made from organic bases including primary, secondary, tertiary amines and substituted amines including cyclic amines such as caffeine, arginine, diethylamine, N-ethyl piperidine, aistidine, glucamine, isopropylamine, lysine, morpholine, N-ethyl morpholine, piperazine, piperidine, triethylamine, trimethylamine. All of the salts contemplated herein may be prepared by conventional means from the corresponding compound by reacting, for example, the appropriate acid or base with the compound.

AMG 900, N-(4-((3-(2-amino-4-pyrimidinyl)-2-pyridinyl)oxy)phenyl)-4-(4-methyl-2-thienyl)-1-phthalazinamine, may be prepared by the procedure analogous to that described in PCT publication WO2007087276, Example Methods A1 or A2 on pg 70 but using 1-chloro-4-(4-methyl-2-thienyl)phthalazine as the starting material, in conjunction with Examples 15 (pg 50), 25 (pg 55) and 30 (pg 59). These procedures are also described in US Patent No. 7,560,551. Specifically, AMG 900 may be prepared as described in Example 1 below.

### Example 1

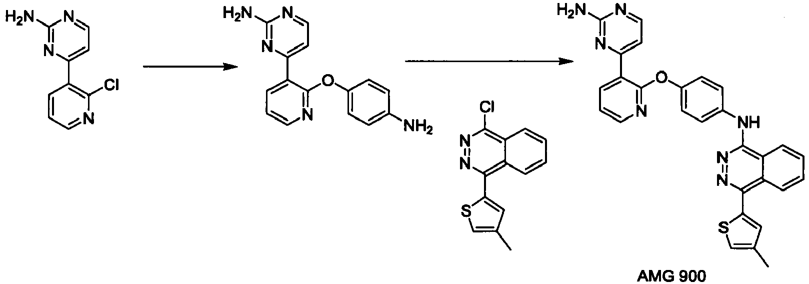

### Synthesis of N-(4-((3-(2-amino-4-pyrimidinyl)-2-pyridinyl)oxy)phenyl)-4-(4-methyl-2-thienyl)-1-phthalazinamine (AMG 900)

### Step 1: 4-(2-chloropyridin-3-yl)pyrimidin-2-amine

In an argon purged 500 mL round bottom flask placed in an isopropanol bath, was added sodium metal (3.40g, 148mmol) slowly to methanol (180mL). The mixture was stirred at room temperature (RT) for about 30 minutes. To this was added guanidine hydrochloride (12.0 mL, 182 mmol) and the mixture was stirred at RT for 30 minutes, followed by addition of (E)-1-(2-chloropyridin-3-yl)-3-(dimethylamino)prop-2-en-1-one (12.0 g, 57.0 mmol), attached air condenser, moved reaction to an oil bath, where it was heated to about 50 °C for 24 h. Approximately half of the methanol was evaporated under reduced pressure and the solids were filtered under vacuum, then washed with saturated sodium bicarbonate (NaHCO₃) and H₂O, air dried to yield 4-(2-chloropyridin-3-yl)pyrimidin-2-amine as off white solid. MS *m*/*z* = 207 [M+1]⁺. Calc'd for C₉H₇ClN₄: 206.63.

### Step 2: 4-(2-(4-aminophenoxy)pyridin-3-yl)pyrimidin-2-amine

To a resealable tube was added 4-aminophenol (1.3 g, 12 mmol), cesium carbonate (7.8 g, 24 mmol), and DMSO (16 ml, 0.75 M). The mixture was heated to 100 °C for 5 minutes, and then 4-(2-chloropyridin-3-yl)pyrimidin-2-amine (2.5 g, 12 mmol) was added, and the reaction mixture was heated to 130 °C overnight. Upon completion, as judged by LCMS, the reaction mixture was allowed to cool to RT and diluted with water. The resulting precipitate was filtered, and the solid washed with water and diethyl ether. The solid was then taken up in 9:1 CH₂Cl₂:MeOH and passed through a pad of silica gel with 9:1 CH₂Cl₂:MeOH as eluent. The solvent was concentrated *in vacuo* to provide the desired product, 4-(2-(4-aminophenoxy)pyridin-3-yl)pyrimidin-2-amine. MS *m*/*z* = 280 [M+1]⁺. Calc'd for C₁₅H₁₃N₅O: 279.30.

### Step 3: 1-Chloro-4-(4-methylthiophen-2-yl)phthalazine

1,4-Dichlorophthalazine (1.40 g, 7.03 mmol), 4-methylthiophen-2-ylboronic acid (999 mg, 7.03 mmol), and PdCl₂(DPPF) (721 mg, 985 µmol) were added into a sealed tube. The tube was purged with Argon. Then sodium carbonate (2.0 M in water) (7.74 ml, 15.5 mmol) and 1,4-dioxane (35.2 ml, 7.03 mmol) were added. The tube was sealed, stirred at RT for 5 min, and placed in a preheated oil bath at 110 °C. After 1 h, LC-MS showed product and byproduct (double coupling), and starting material dichlorophthalazine. The reaction was cooled to RT, filtered through a pad of celite with an aid of ethyl acetate (EtOAc), concentrated, and loaded onto column. The product was purified by column chromatography using Hex to remove the top spot, then 80:20 hexanes:EtOAc to collect the product. The product, 1-chloro-4-(4-methylthiophen-2-yl)phthalazine was obtained as yellow solid. LC-MS showed that the product was contaminated with a small amount of dichlorophthalazine and biscoupling byproduct. MS m/z = 261 [M+1]⁺. Calcd for C₁₃H₉ClN₂S: 260.12.

### Step 4: N-((3-(2-amino-4-pyrimidinyl)-2-pyridinyl)oxy)phenyl)-4-(4-methyl-2-thienyl)-1-phthalazinamine

To 4-(2-(4-aminophenoxy)pyridin-3-yl)pyrimidin-2-amine and 1-chloro-4-(4-methyl-2-thienyl)phthalazine was added tBuOH. The resulting mixture was heated at 100 °C in a sealed tube for 16 hours. The rection was diluted with diethyl ether and saturated sodium carbonate and vigorously shaken. The resulting solids were filtered and washed with water, diethyl ether and air dried to yield N-(4-((3-(2-amino-4-pyrimidinyl)-2-pyridinyl)oxy)phenyl)-4-(4-methyl-2-thienyl)-1-phthalazinamine as an off-white solid. MS *m*/*z* = 504 [M+H]⁺. Calc'd for C₂₈H₂₁N₇OS: 503.58.

### LC-MS Method:

Samples were run on a Agilent model-1100 LC-MSD system with an Agilent Technologies XDB-C₈ (3.5 µ) reverse phase column (4.6 x 75 mm) at 30 °C. The flow rate was constant and ranged from about 0.75 mL/min to about 1.0 mL/min.

The mobile phase used a mixture of solvent A (H₂O/0.1% HOAc) and solvent B (AcCN/0.1% HOAc) with a 9 min time period for a gradient from 10% to 90% solvent B. The gradient was followed by a 0.5 min period to return to 10% solvent B and a 2.5 min 10% solvent B re-equilibration (flush) of the column.

Other methods may also be used to synthesize AMG 900. Many synthetic chemistry transformations, as well as protecting group methodologies, useful in synthesizing AMG 900, are known in the art. Useful organic chemical transformation literature includes, for example, R. Larock, Comprehensive Organic Transformations, VCH Publishers (1989); T.W. Greene and P.G.M. Wuts, Protective Groups in Organic Synthesis, 3rd edition, John Wiley and Sons (1999); L. Fieser and M. Fieser, Fieser and Fieser's Reagents for Organic Synthesis, John Wiley and Sons (1994); A. Katritzky and A. Pozharski, Handbook of Heterocyclic Chemistry, 2nd edition (2001); M. Bodanszky, A. Bodanszky, The Practice of Peptide Synthesis, Springer-Verlag, Berlin Heidelberg (1984); J. Seyden-Penne, Reductions by the Alumino- and Borohydrides in Organic Synthesis, 2nd edition, Wiley-VCH, (1997); and L. Paquette, editor, Encyclopedia of Reagents for Organic Synthesis, John Wiley and Sons (1995).

AMG 900 was tested for its ability to reduce or inhibit tumor progression in various cell lines (*in-vitro*) and multiple solid tumor types (*in-vivo*), some of which have previously been exposed to and developed resistance to standard-of-care antimitotic agents, including taxanes and vinca alkaloids, as well as to other chemotherapeutic agents. The following Examples and resulting data will illustrate the ability of AMG 900 to treat cancer, including cancer resistant to the presently standard-of-care therapies, including antimitotic agents, such as paclitaxel, and other drugs used in conjunction with chemotherapy, such as doxorubicin. Unless otherwise indicated, the free base form of AMG 900 was used in the Examples described hereinbelow.

### Example 2

To investigate whether AMG 900-induced suppression of aurora kinase A and B activity inhibits cell proliferation, the antiproliferative effect of AMG 900 was evaluated *in vitro* using 32 human tumor cell lines. As shown in Table 1 and Table 2, AMG 900 exhibited antiproliferative activity across both solid and hematologic tumor cell lines. This antiproliferative activity was seen with concentrations of AMG 900 in the low nanomolar range (ECso values 1 to 5 nM). Importantly, four of these AMG 900-sensitive solid tumor cell lines (HCT15, MES-SA Dx5, 769P, and SNU449) are resistant to paclitaxel and other chemotherapeutic agents. Cancer cells resistant to multiple drugs of different chemical structures and/or resistant to drugs directed at different targets are termed "multidrug resistant". One prominent mechanism of multidrug resistance (MDR) utilized by cancer cells is drug efflux mediated by a family of ATP-binding cassette (ABC) transporters, such as the *mdr-1* gene product, P-glycoprotein (P-gp). For example, the doxorubicin-resistant human uterine cell line MES-SA Dx5, expresses P-gp and is resistant (30- to 1200-fold over parent line) to a number of chemotherapeutic agents including daunorubicin, dactinomycin, colchicine, vinblastine, vincristine, paclitaxel, etoposide, and mitoxantrone. To futher investigate the activity of AMG 900 in MDR-expressing cells, three taxol-resistant tumor cell lines were tested and compared to their respective parental cell lines. As shown in Figures 1-3, and Table 3, AMG 900 maintained potency in all three matched taxol-resistant and -sensitive tumor cell lines with EC₅₀ values < 2 nM. Taxol showed a significant loss of potency (10- to 100-fold) in the P-gp expressing tumor sublines compared to the parental lines. Together these data indicate that AMG 900 inhibits phosphorylation histone H3 (a proximal substrate of aurora kinase B) and blocks cell division of tumor cell lines resistant to paclitaxel and other chemotherapeutic agents.

### MATERIALS AND METHODS

**Test Materials**

| | |
|---|---|
| Test article: | AMG 900 |
| Formulation: | DMSO |
| Source: | Amgen Inc. |

### Critical Reagents

**Wash and Fixation Solutions**

| | |
|---|---|
| 1X PBS | Dulbecco's phosphate buffer saline, Cat# 14090-144, Invitrogen Corp., Carlsbad, CA 92008 |
| Distilled (d) H₂0 | Cat# 2F7115, Baxter Health care Corp., Deerfield, IL 60015 |
| 90% Methanol in dH20 | Methyl Alcohol, Cat# 3041-10, Mallinckrodt Chemicals, Phillipsburg, NJ 08865 |
| **Wash Buffer (in 1XPBS)** - **FACS analysis** | |
| 1% BSA | Cat# 810111, Qty 50 mL, ICN Biomedicals Inc. Aurora, OH 44202 |
| 0.2% Triton X-100 | Cat# 9284, Sigma-Aldrich, St. Louis, MO 63178 |
| **Wash Buffer - Cellomics** 1X PBS | Dulbecco's phosphate buffer saline, Cat# 14090-144, Invitrogen Corp., Carlsbad, CA 92008 |
| 1% normal goat serum | Cat# G-9023-10 mL, Sigma-Aldrich, St. Louis, MO |
| 1% Tween-20 | 63178 |
| | Cat#P-1379, Sigma-Aldrich, St. Louis, MO 63178 |
| **Acid Buffer(in dH₂0)** | Hydrochloric Acid 37 %, Cat# JT953000, Lot# not available, Sigma-Aldrich, St. Louis, MO 63178 |
| 2N HCL | |
| 0.5% Triton X-100 | Cat# 9284, Lot# not available, 500 mL, Sigma-Aldrich, St. Louis, MO 63178 |
| **2X Formaldehyde Fixation (in 1X PBS)** - **Cellomics** | Cat#F1635-500mL, Sigma-Aldrich, St. Louis, MO 63178 |
| 20% formaldehyde, 0.024% glutaraldehyde | |
| | Cat# 85191, Fluka/Sigma-Aldrich, St. Louis, MO 63178 |

**Reagents**

| | |
|---|---|
| Propidium Iodide/RNAse Staining Buffer | PI/RNAse, Cat# 550825, 100mL, Becton Dickinson, San Jose, CA 95131 |
| DMSO, dimethyl sulphoxide | Cat# D2650, Sigma-Aldrich, St. Louis, MO 63178 |
| Anti-phospho-Histone H3 (Ser10) antibody | Cat# 06-570, Upstate Cell Signaling Solutions, Lake Placid, NY 12946 |
| (p-histone H3), mitosis marker | |
| Alexa Flour 488 goat anti-rabbit IgG antibody | Cat # A11001, Invitrogen Corp., Carlsbad, CA 92008 |
| Hoechst 33342 trihydrochloride, trihydrate | Cat# H3570, Invitrogen Corp., Carlsbad, CA 92008 |
| Anti-bromodeoxyuridine, mouse IgG1, monoclonal PRB-1, Alexa Fluor 647 conjugate | Cat# A21305, Lot#54656A, Invitrogen Corp.Carlsbad, CA 92008 |
| (anti-BrdU, Alexa-647) | |
| FITC-conjugated rabbit anti-active Caspase-3 monoclonal antibody (anti-Caspase-FITC) | 100 tests, Cat# 559341, Lot# 60509, BD Pharmingen, San Diego, CA 92121 |
| BrdU labeling reagent (stock concentration not specified) | Cat#00-0103, Zymed Laboratories, Carlsbad, CA 92008 |
| 1X Trypsin-EDTA, 0.5% | Cat# 25300-054, Invirtogen Corp., Carlsbad, CA 92008 |
| 1X Versene | Cat# 15040-066, Invirtogen Corp., Carlsbad, CA 92008 |
| McCoys 5A medium, (+)L-Glut | Cat# 16600-082 Invitrogen Corp., Carlsbad, CA 92008 |
| RPMI medium 1640 (+) L-Glut. | Cat# 11875-093, Invitrogen Corp., Carlsbad, CA 92008 |
| DMEM, high glucose, (+) 4.5 g/L D-glucose, (+)L-Glut. | Cat# 11965-092 Invitrogen Corp., Carlsbad, CA 92008 |
| FBS, fetal bovine serum, origin - Australia | Cat# 10099-141, Invitrogen Corp., Carlsbad, CA 92008 |
| Taxol (Paclitaxel) | Cat# T7402, Sigma-Aldrich, St. Louis, MO 63178 |
| Vinblastine | Cat# V1377, Sigma-Aldrich, St. Louis, MO 63178 |

**Lab Equipment, Supplies, Software**

| Table top refrigerated centrifuge | Allegra X-15R Centrifuge, Beckman Coulter, Fullerton, CA 92834 |
|---|---|
| GraFit v5 software | Erithacus Software, Horley Surrey, RH6 9YJ, UK |
| XLfit 4.2 software | Excel, Microsoft Inc., USA |
| GraphPad Prism v5 | GraphPad Software, Inc. 2236 Avenida de la Playa, La Jolla, CA 92037 |
| Flow Cytometer | Becton Dickinson LSRII Flow Cytometer, BD Biosciences, San Jose, CA 95131 |
| 96-well tissue culture plate, flat bottom with low evaporation lid, sterile | Cat# 3595, Lot # not available, Corning Incorp. Life Sciences, Lowell MA 01851 |
| 12-well tissue culture plate, flat bottom with low evaporation lid, sterile | Cat# 353043, Lot # not available, BD Labware, Franklin Lakes, NJ 07417 |
| 6-well tissue culture plate, flat bottom with low evaporation lid, sterile | Cat# 353046, Lot # not available, BD Labware, Franklin Lakes, NJ 07417 |
| PCR tube Strips used for FACS staining | Cat# 20170-004, Lot# 711C7-7074, 0.2 mL strip tube for PCR, VWR Intl., West Chester, PA 19380. |
| 1.5mL Eppendorf Tubes | Cat# 20901-551, Lot# not available, VWR Intl., West Chester, PA 19380 |
| Packard View-96 Plates | Cat#-6005182, Lot# Not available, PerkinElmer Life and Analytical Sciences, Waltham, MA 02451 |
| ELX405 plate washer | Cat# ELX405HT, BioTek, Winooski, VT 05404 |
| Multidrop DW(Deep Well) | Cat# 5840177, Thermo Fisher Scientific Inc, Waltham, MA 02454 |
| Cellomics Array Scan VTi | Cellomics-Thermo Fisher Inc, Waltham, MA 02454 |

### Methods

The activity of AMG 900 was assessed *in vitro* on parental and drug-resistant tumor cell lines derived from uterine, breast, and lung tissues. Cell-cycle and p-Histone H3 endpoints were assessed by flow cytometry and high-content cell imaging, respectively.

### Human Cell and Cell Culture

Human tumor cell lines were obtained from the American Type Culture Collection (Manassas, VA) unless otherwise indicated. All cells are maintained in at 37 degrees Celsius in an atmosphere of 5% CO₂. The breast tumor derived CAL51 (ACC 302) cell line was obtained from DSMZ (GmbH). The colon tumor derived HCT-116_JH (genotype p21+/+) and HCT-116_JH (genotype p21-/-) cell lines were obtained under license from John Hopkins University Genetics Resources Core Facility.

**Human Tumor Cell Lines with Corresponding Culture Media**

| **Tumor cell line** | **Origin** | **Tissue culture growth conditions** |
|---|---|---|
| | | (all media contains 1x glutamine) |
| HCT-116_JH (genotype p21 +/+) | Colon | McCoy's 5A, 10% FBS + L-glutamine |
| HCT-116_JH (genotype p21 -/-) | Colon | McCoy's 5A, 10% FBS + L-glutamine |
| HCT-15 | Colon | RPMI 1640, 10% FBS + L-glutamine |
| HT29 | Colon | McCoy's 5A, 10% FBS + L-glutamine |
| SW-620 | Colon | RPMI 1640, 10% FBS + L-glutamine |
| SW480 | Colon | RPMI 1640, 10% FBS + L-glutamine |
| HOP-92 | Lung | RPMI 1640, 10% FBS + L-glutamine |
| HOP-62 | Lung | RPMI 1640, 10% FBS + L-glutamine |
| NCI-H460 | Lung | RPMI 1640, 10% FBS + L-glutamine |
| A549 | Lung | Ham's F12K+10% FBS+ L-glutamine |
| PC-3 | Prostate | RPMI 1640, 10% FBS + L-glutamine |
| DU-145 | Prostate | RPMI 1640, 10% FBS + L-glutamine |
| BT-549 | Breast | RPMI 1640, 10% FBS + L-glutamine |
| BT-474 | Breast | RPMI 1640, 10% FBS + L-glutamine |
| MDA-MB-231 | Breast | RPMI 1640, 10% FBS + L-glutamine |
| T47D | Breast | RPMI 1640, 10% FBS + L-glutamine |
| MCF-7 p53(+) | Breast | RPMI 1640, 10% FBS + L-glutamine |
| MCF-7 p53(-) | Breast | RPMI 1640, 10% FBS + L-glutamine |
| CAL51 | Breast | DMEM+ +10% FBS+ 1XNEAA + L-glutamine |
| SK-OV-3 | Ovarian | McCoy's 5A, 10% FBS + L-glutamine |
| MES-SA/Dx5 | Uterine | McCoy's 5A, 10% FBS + L-glutamine |
| MES-SA | Uterine | McCoy's 5A, 10% FBS |
| SK-MEL-2 | Skin | MEM, 10% FBS, ImM Na Pyruvate, 0.1mMNEAA + L-glutamine |
| A498 | Renal | MEM, 10% FBS, 1x NEAA + L-glutamine |
| 769P | Renal | RPMI 1640, 10% FBS + L-glutamine |
| CAKI-1 | Renal | McCoy's 5A, 10% FBS + L-glutamine |
| SK-HEP-1 | Liver | MEM, 10% FBS, 1x NEAA + L-glutamine |
| SNU449 | | RPMI 1640, 10% FBS, 10 mM HEPES, 1mM Na |
| | Liver | Pyruvate + L-glutamine |
| K562 | Leukemia | RPMI 1640, 10% FBS + L-glutamine |
| MOLT-4 | Leukemia | RPMI 1640, 10% FBS + L-glutamine |
| HL-60 | Leukemia | RPMI 1640, 10% FBS + L-glutamine |
| Jurkat | Leukemia | RPMI 1640, 10% FBS + L-glutamine |
| U266-B1 | Myeloma | RPMI 1640, 15% FBS + L-glutamine |
| RPMI-8226 | Myeloma | RPMI 1640, 20% FBS + L-glutamine |
| NCI-H460 taxol-r | Lung | RPMI 1640, 10% FBS, 75 nM Taxol |
| MDA-MB-231(F11)-luc | Breast | DMEM, 10% FBS |
| MDA-MB-231(F11)-luc taxol-r | Breast | DMEM, 10% FBS, 50 nM Taxol |

### Panel of Solid Tumor Cell Line Treated with AMG 900: Antiproliferative Assay (ArrayScan VTi)

Tumor cells were seeded in a Packard View 96-well plate in 100 µL of appropriate complete media at a density of 3000 or 5000 cells/well depending on the cell line growth kinetics (broadly defined as slow vs. fast). All dilutions were performed using Biomek FX workstation. The next day cells were treated with AMG 900 (11-point dose range 0.156 to 0.0003 µM) with a final DMSO concentration of 0.12% in media. After 24 hours, the media containing compound was removed and the cells were washed with complete media. The cells were then incubated in 100 µL of fresh complete media (without compound) for 48 hours. After 48 hours, the cells were fixed by adding 100 µL of fixation buffer to 100 µL of complete media. The cells were incubated at room temperature for 10 minutes. The fixation buffer was aspirated and the cells were then permeabilized in 100 µL wash buffer for 30 minutes at room temperature followed by the addition of 100 µL of DNA stain buffer and incubated at room temperature for 30 minutes in the dark. Next, the cells were washed with 100 µL of wash buffer and stored in 100 µL 1x PBS at 4 degrees Celsius until analysis. Cellular data was attained by scanning the 96-well plates on an ArrayScan VTi imaging system (Cellomics). Quantification of individual cell nuclear area and intensity was performed based on Hoechst 33342 DNA dye fluorescence. A threshold on the nuclear area based on the DMSO-treated control well was set to quantify the number of normal size nuclei from nuclear debris and polyploidy cells. This threshold value was applied to enumerate the number of normal nuclei in six image fields/well using a magnification of 10x. The dose-concentration curves and ECso values were calculated using a 4-parameter equation.

### Panel of Hematologic Cell Line Treated with AMG 900: Multiparameter Cell Cycle DNA Content Assay (Flow Cytometry)

Tumor cells were seeded in a 300 µL deep 96-well plate in 150 µL of appropriate complete media at a density of 100,000 cells/well. The cells were treated with AMG 900 (10-point dose range 0.156 to 0.0003 µM) with a final DMSO concentration of 0.2% in media. Two hours prior to harvest time, the cells were pulsed with BrdU at the final concentration of 1:100 in media. After 48 hours, 100 µL of media was removed from each well with a multi-well pipetter. Then the cells were transferred to PCR tube strips in 200 µL of media. The cells were centrifuged at 2000 rpm at 18°C for 4 minutes and the supernatant was aspirated. The cells were then fixed in 200 µL of ice cold 90% methanol and stored at -20°C for at least 24 hours before antibody and DNA staining. Fixed cells were centrifuged at 2000 rpm for 4 minutes to remove 90% methanol. Cells were washed with 200 µL of wash buffer and then treated with 100 µL acid buffer at room temperature for 1 hour in the dark. Cells were washed twice with 200 µL wash buffer (until pH was 7.0, confirmed with pH paper). Cells were incubated with an anti-BrdU-Alexa 647 (3 µg/mL) and anti-Caspase 3-FITC (20 µl/well) antibody cocktail in wash buffer for 2 hours at room temperature in the dark. Stained cells were centrifuged and washed with 200 µL of wash buffer. Cells were counterstained with 200 µL of propidium iodide (PI) overnight at 4° C in the dark. Data was acquired and analyzed by flow cytometer (LSRII). A threshold gate was applied according to DNA content, BrdU, and Caspase-3 positive/negative populations based on DMSO control and low/high drug-treated groups. The data was represented as a percentage of SubG1 DNA content, 4N+ DNA content, BrdU, and Caspase-3 cleavage positive populations. The concentration-response curves and EC₅₀ values were calculated using a 4-parameter equation.

### MES-SA Dx5 and MES-SA Cell Lines Treated with AMG 900 or Paclitaxel (taxol): p-Histone H3 Assay (ArrayScan VTi)

MES-SA Dx5 and MES-SA cells were plated at a density of 10,000 cells/well onto 96-well plate in complete media and cultured for 24 hours. The next day cells were treated with AMG 900 or taxol over a 10-point concentration range (1.25 µM to 0.0024 µM) for 24 hours with a final DMSO concentration of 0.1% in media. Cells were washed and fixed by adding 100 µL of 2x formaldehyde fixation buffer for 10 minutes and room temperature. Cells were washed and permeabilized with 100 µL of wash buffer for 15 minutes. Cells were immunostained with p-histone H3 antibody (5 µg/mL) and incubated at room temperature for 2 hours. Cells were washed in 100 µL of wash buffer. Next, cells were incubated with a goat anti-rabbit alexa-488 conjugated antibody (1.5 µg/mL) in wash buffer supplemented with Hoechest DNA dye (1 µg/mL) and incubated at room temperature in the dark for 30 minutes. Cells were washed twice with 100 µL of wash buffer. The 96-well plates were analyzed on an ArrayScan VTi (Cellomics) using a bioapplication algorithm (TargetActivation.V2). The percentage of p-histone H3+ objects (summation of 6 image fields/well with a 10x objective) were used to generate concentration-response curves and EC₅₀ values using a 4-parameter equation.

### NCI-H460 parent and Taxol-resistant cell lines treated with AMG 900 or Paclitaxel (taxol): Cell Cycle DNA Content Assay (Flow Cytometry)

NCI-H460 parental and NCI-H460 taxol-resistant cells were seeded at a density of 500,000 cells/well in a 6-well plate in 2 mL of appropriate complete media. The next day cells were treated with AMG 900 or taxol over a 6-point concentration range with a final DMSO concentration of 0.05% in media. After 24 hours, cells were pulsed with BrdU (1:100 dilution) and harvested. Cells were centrifuged at 1600 rpm for 4 minutes and the supernatant was aspirated. Cells were washed in 1x PBS and fixed in 900 µL of ice cold 90% methanol and stored at -20°C. Fixed cells were centrifuged at 2000 rpm for 4 minutes to remove 90% methanol. Cells were washed with 200 µL of wash buffer and stained with 200 µL of propidium iodide (PI) overnight at 4°C in the dark. Data was acquired and analyzed by flow cytomety (LSRII). A threshold gate was applied according to +4N (same as ≥ 4N) DNA content and SubG1 positive populations based on DMSO-treated control and low/high drug-treated groups. The data was represented as a percentage of +4N DNA and SubG1 positive subpopulations. The +4N (same as ≥ 4N) DNA content or SubG1 cellular EC₅₀ values were calculated using a 4-parameter equation.

### MDA-MB-231 (F11)-luc parent and Taxol-resistant cell lines treated with AMG 900 or Paclitaxel (taxol): Cell Cycle DNA Content Assay (Flow Cytometry)

MDA-MB-231 (F11)-luc parent and taxol-resistant cells were seeded at a density of 250,000 cells/well in a 12-well plate in 1 mL of in the appropriate media, in duplicates. The next day cells were treated with AMG 900 or taxol over a 10-point concentration range with a final DMSO concentration of 0.1% in taxol-free media. After 24 hours, cells were harvested with 1x trypsin-EDTA and transferred to PCR tubes. Cells were centrifuged at 2000 rpm for 4 minutes and the supernatant was aspirated. Cells were fixed in 200 µL of ice cold 90% methanol and stored at -20°C for at least 24 hours. Fixed cells were centrifuged at 2000 rpm for 4 minutes to remove 90% methanol. Cells were washed with wash buffer and stained with 200 µL of propidium iodide (PI) for 30 minutes at 4°C in the dark. An additional 400 µL of PI was added before data acquisition. Data was acquired and analyzed by flow cytometry (LSRII). A threshold gate was applied according to +4N (same as ≥4N) DNA content based on DMSO control and low/high drug-treated groups. The data was represented as percentage of control for +4N DNA content positive populations. The +4N DNA content cellular EC₅₀ values were assigned using 4-parameter equation.

**Table 1. In Vitro AMG 900 Inhibits Cell Proliferation of Multiple Solid Tumor Types**

| **Tumor Cell Line** | **Origin** | **AMG 900 ECso (µM)** |
|---|---|---|
| HCT 116_JH_p21-/- | Colon | 0.001 |
| HCT 116_JH_p21+/+ | Colon | 0.001 |
| HCT15* | Colon | 0.002 |
| HT29 | Colon | 0.005 |
| SW620 | Colon | 0.002 |
| SW480 | Colon | 0.002 |
| HOP-92 | Lung | 0.002 |
| HOP-62 | Lung | 0.002 |
| NCI-H460 | Lung | 0.001 |
| A549 | Lung | 0.001 |
| PC3 | Prostate | 0.002 |
| DU145 | Prostate | 0.002 |
| BT549 | Breast | 0.004 |
| MDA-MB-231 | Breast | 0.002 |
| T47D | Breast | 0.005 |
| MCF7-p53+ | Breast | 0.001 |
| MCF7-p53- | Breast | 0.003 |
| CAL51 | Breast | 0.002 |
| SK-OV-3 | Ovarian | 0.002 |
| MES-SA Dx5* | Uterine | 0.001 |
| SK-MEL-2 | Skin | 0.002 |
| A498 | Kidney | 0.001 |
| 769P* | Kidney | 0.002 |
| CAKI-1 | Kidney | 0.001 |
| SK-HEP-1 | Liver | 0.001 |
| SNU449* | Liver | 0.002 |

| | | |
|---|---|---|
| *Paclitaxel-resistant tumor cell lines (Gyorffy et al, 2006; Harker, G.A. et al. Multidrug (Pleiotropic) Resistance in Doxorubicin-selected Variants of Human Sarcoma Cell Line MES-SA. Cancer Research 1985: 45: 4091-4096; Szakacs, G. et al. Predicting drug sensitivity and resistance: Profiling ABC transporter genes in cancer cells. Cancer Cell 2004: 6: 129-137; Szakacs, G. et al. Targeting multi-drug resistance in cancer. Nat. Rev. Drug Discovery 2006: 5: 219-234 | | |

**Table 2. In Vitro AMG 900 Blocks Cell Division in Several Hematologic Tumor Types**

| **Tumor Cell Line** | **Hematologic Type** | **AMG 900 EC₅₀ (µM)** |
|---|---|---|
| HL-60 | Promyelocytic leukemia | 0.002 |
| K562 | Chronic myeloid leukemia | 0.001 |
| MOLT-4 | T cell leukemia | 0.002 |
| Jurkat | T cell leukemia | 0.001 |
| RPMI-8226 | Multiple Myeloma | 0.002 |
| U266-B 1 | Multiple Myeloma | 0.002 |

| | | |
|---|---|---|
| Cells were treated with AMG 900 for 48 hours (without compound withdrawal). Flow cytometry-based analysis was performed using multiple endpoints (caspase-3 cleavage, BrdU, SubGl, and +4N DNA content (≥4N DNA content)). EC₅₀ values were generated using +4N DNA content endpoint. Reported EC₅₀ values represent single 10-point dose-response curve. | | |

**Table 3. In Vitro AMG 900 Maintains Potency in Tumor Cell Lines Resistant to Paclitaxel**

| **Origin** | **Tumor Cell Line** | | **AMG 900 EC₅₀ (µM) @ 24 hours** | **Paclitaxel EC₅₀ (µM) @ 24 hours** |
|---|---|---|---|---|
| Uterine^{a} | MES-SA | Parent line | <0.002 | 0.01 |
| | MES-SA Dx5¹ | Doxorubicin | <0.002 | >1.25 |
| Breast^{b} | MDA-MB-231 | Parent line | 0.001 | 0.002 |
| | MDA-MB-231-Taxol-r² | Paclitaxel | 0.001 | 0.095 |
| Lung^{c} | NCI-H460 | Parent line | 0.001 | 0.01 |
| | NCI-H460-Taxol-r² | Paclitaxel | 0.001 | >0.1 |

| | | | | |
|---|---|---|---|---|
| Resistance (r), defined as a ≥10-fold loss of potency (EC₅₀ value) in the subline compared to the parental line. "Cellomics-based p-Histone H3 analysis. Reported EC₅₀ values represent a single experiment with 10-point dose response. ^{b}Flow cytometry-based DNA content analysis. EC₅₀ values were generated with appropriate DNA content endpoint (≥4N DNA content (AMG 900) and Sub G₁ (paclitaxel)). EC₅₀ values represent a single experiment performed in duplicate with 10-point dose response. MDA-MB-231 cell lines contain a transgene expressing both green fluorescent protein and luciferase protein. ^{c}Flow cytometry-based DNA content analysis. EC₅₀ values were generated with appropriate DNA content endpoint (≥4N DNA content (AMG 900) and Sub G₁ (paclitaxel)). EC₅₀ values represent a single experiment performed with 6-point dose response. ¹The multiple drug resistant subline, MES-SA Dx5, was established from parental MES-SA cell line by growing cells in the presence of increasing concentrations of doxorubicin. The MES-SA Dx5 cell line overexpresses P-gp (Harker et al, 1985). ²The paclitaxel-resistant sublines MDA-MB-231-Taxol-r and NCI-H460-Taxol-r were derived from their respective parental line by growing cells in the presence of increasing concentrations of paclitaxel. Both sublines are positive for P-gp by flow cytometry. | | | | |

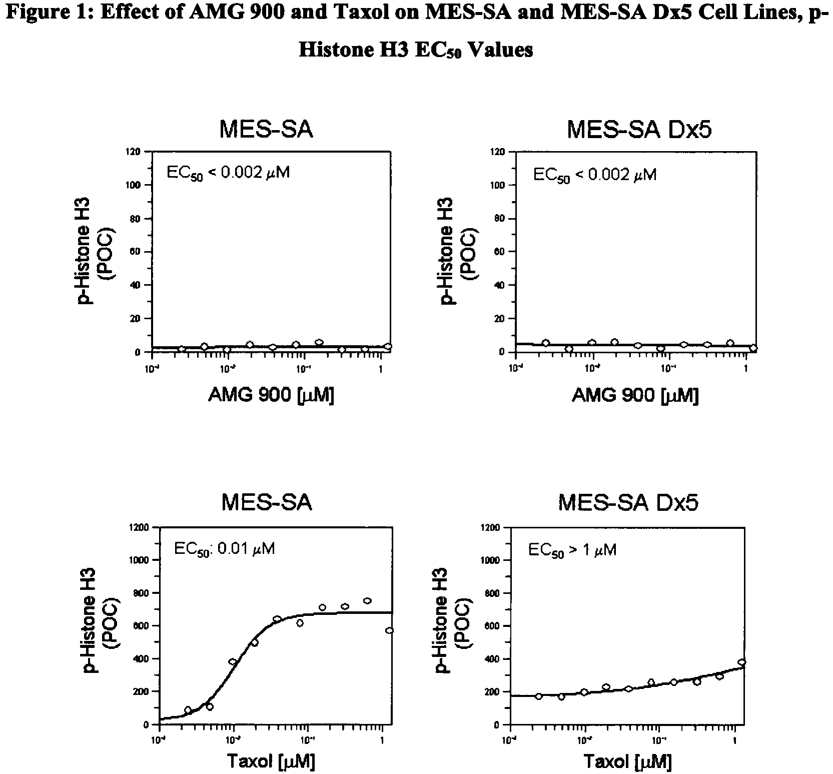

Uterine tumor cell lines were treated with control (DMSO), AMG 900 or paclitaxel (taxol) over a 10-point dose range (0.0024□ to 1.25 µM□) for 24 hours. Cells were then fixed and stained with p-histone H3 antibody and counterstained with a DNA dye (Hoechest). Imaging-based analysis (ArrayScan VTi) was performed to measure the percentage of p-histone H3 positive cells. The dose-response curves represent either AMG 900 or taxol concentrations plotted against p-Histone H3 positive cells as a percentage of DMSO-treated control (POC). The EC₅₀ values were calcaluted by 4-parameter fit model.

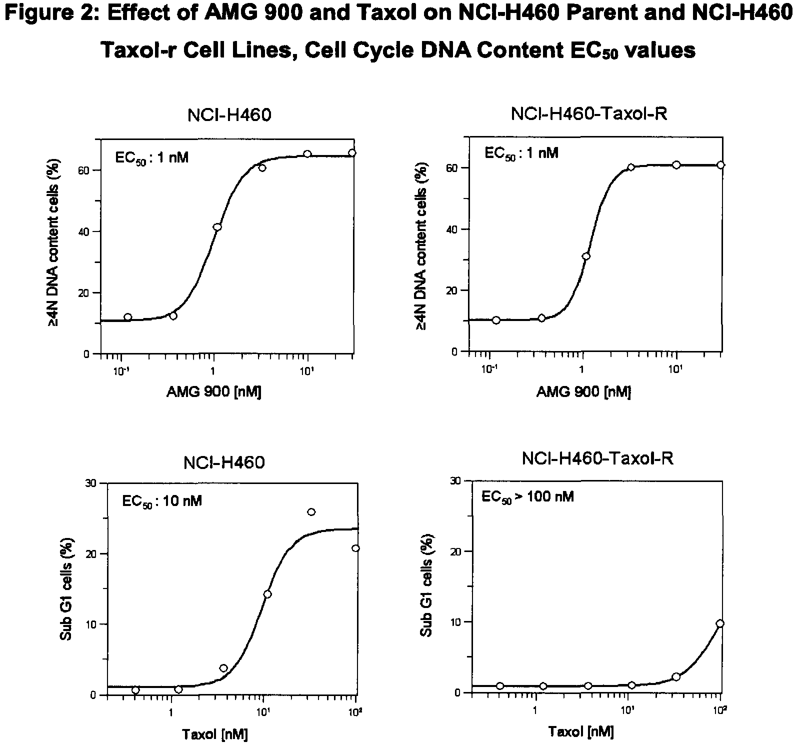

Lung tumor cell lines were treated with control (DMSO), AMG 900 or taxol over a 6-point dose range for 24 hours. Flow cytometric-based cell cycle analysis was performed to measure the percentage of ≥4N DNA content or SubG1 DNA content positive cells. The dose-response curves for AMG 900 represent the drug concentration plotted against the percentage of ≥4N DNA content positive cells. The dose-response curves for taxol represent the drug concentration plotted against the percentage of SubG1 DNA content positive cells. The EC₅₀ values were calculated by 4-parameter fit model.

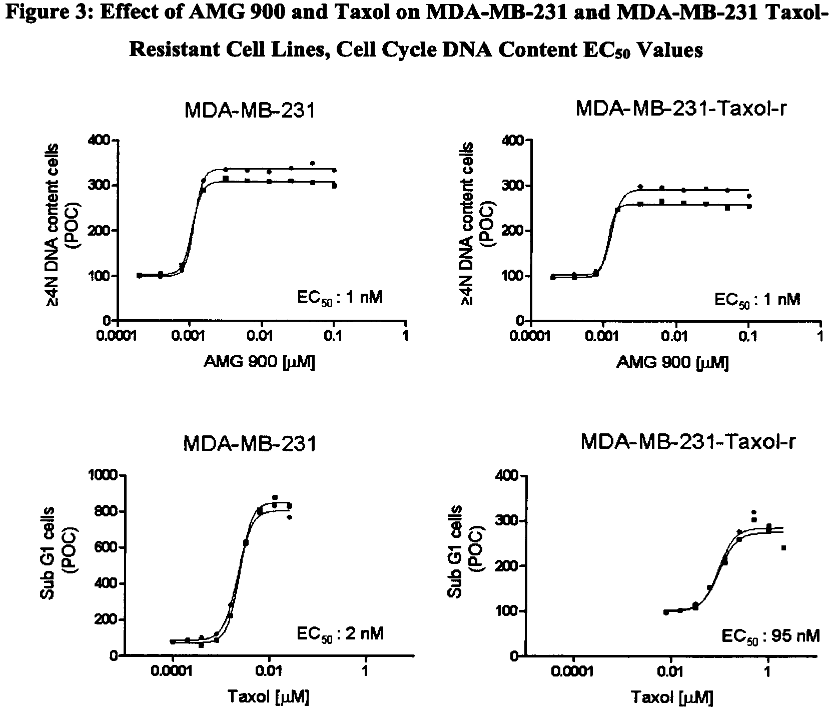

Breast tumor cell lines were treated with control (DMSO), AMG 900 or taxol over a 10-point dose range for 24 hours. Flow cytometric-based cell cycle analysis was performed to measure the percentage of ≥4N DNA content or SubG1 DNA content positive cells. The dose-response curves for AMG 900 represent the drug concentration plotted against the percentage of ≥4N DNA content positive cells. The dose-response curves for taxol represent the drug concentration plotted against the percentage of SubG1 DNA content positive cells. The EC₅₀ values were calculated by 4-parameter fit model.

### Example 3

To investigate whether AMG 900-induced suppression of aurora kinase activity inhibits cell proliferation, the antiproliferative efficacy of AMG 900 was evaluated *in-vivo* in multiple human cancer xenograft models, including breast, colon, leukemia, lung, pancreatic, and uterine cancer models, grown in athymic nude mice. Mice were administered AMG 900 orally at 3.75, 7.5, or 15 mg/kg BID for 2 consecutive days per week or 3 mg/kg BID everyday for the duration of the study beginning when tumors were established. The reagents, solutions, equipment, formulation of AMG 900, tumor volume measurements and calculations were generally as described in Example 4 below.
AMG 900 was found to significantly inhibited tumor growth in all xenograft models tested compared with the vehicle control group (Table 4).

**Table 4. AMG 900 Inhibits the Growth of Multiple Xenograft Models**

| Paclitaxel sensitive *in vitro* | Tumor Origin | Cell Line | AMG 900 %TGI* |
|---|---|---|---|
| yes | Colon | HCT 116 | 83 |
| no | Colon | HCT15 | 51 |
| yes | Colon | Colo 205 | 58 |
| yes | Lung | NCI-H460 | 85 |
| no | Lung | NCI-H460-Taxol-r | 66 |
| no | Uterine | MES-SA Dx5 | 73 |
| yes | Uterine | MES-SA | 86 |
| yes | Leukemia | HL60 | 68 |
| yes | Breast | MDA-MB-231 | 82 |
| ND | Pancreas | MiaPaCa2 | 62 |

| | | | |
|---|---|---|---|
| (TGI) tumor growth inhibition, *%TGI picked from either intermittent or continuous dose schedule based on best efficacy response. (ND) Not determined, Resistance (r), defined as a ≥10-fold loss of potency (EC50 value) compared to taxane-sensitive tumor cell lines | | | |

The effect of AMG 900 was tested on the multidrug resistant cell line MES-SA Dx5 grown in vivo as a tumor xenograft. Mice were administered AMG 900 orally at 15 mg/kg BID for 2 consecutive days per week or 3.0 mg/kg BID everyday for the duration of the study. Dosing was initiated when tumors were established (10 days after tumor implantation). AMG 900 treatment resulted in statistically significant tumor growth inhibition using both doses and schedules of AMG 900 compared with the vehicle control group (Figure 4; p < 0.0001, Dunnett's post hoc test). Comparable tumor growth inhibition was also surprisingly achieved in 2 other drug-resistant models (HCT15 and NCI-H460-Taxol-r [resistant]) using similar treatment schedules (See Table 4).

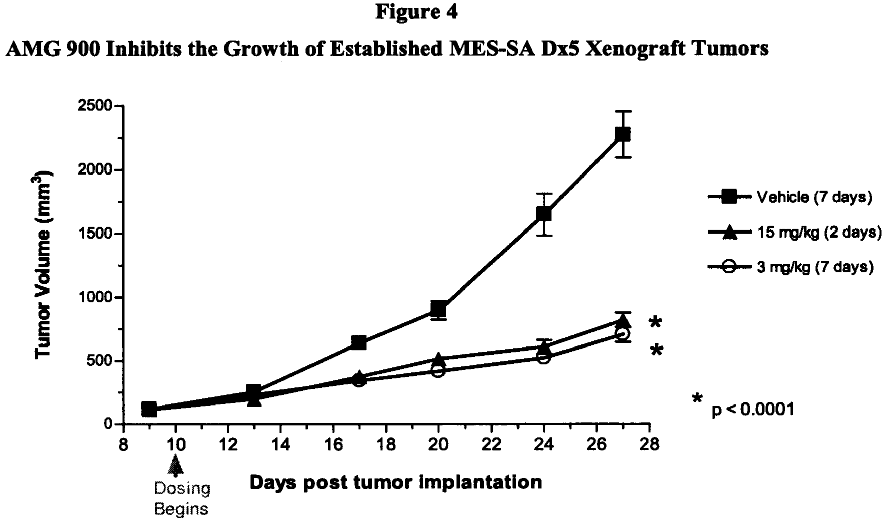

MES-SA Dx5, multi-drug resistant, cells (2 x 10⁶) were injected subcutaneously in the right flank of female athymic nude mice. Tumors were measured twice per week. Treatment began on day 10 when the tumors were ∼100 mm³. Mice were dosed PO with AMG 900 (BID) either intermittently or continuously. All groups were provided nutritional supplements on a daily basis throughout the study to maintain body weight. Data represent mean ± SEM for each group (n = 10 per group). P-values correspond to statistical difference between groups treated with vehicle and AMG 900 determined by RMANOVA followed by Dunnett's post-hoc test. Arrow denotes start of dosing.

### Example 4

To investigate whether AMG 900-induced suppression of aurora kinase activity inhibits cell proliferation, the antitumor efficacy of AMG 900 was evaluated *in vivo* against NCI-H460-taxol resistant tumor xenografts in athymic nude mice. Mice were dosed PO with AMG 900 (BID) either intermittently or continuously. Mice were dosed IP with paclitaxel (taxol) 5 days/week. An internal Amgen compound was used as a positive control in this study (data not shown). Tumors were measured twice per week. Treatment began on day 12 when the tumors were established. All groups were provided nutritional supplements on a daily basis throughout the study to maintain body weight.

**Animals and Tissues**

| | |
|---|---|
| Species/Strain: | Athymic Nude |
| Number/Sex: | 125/female |
| Mean Weight: | 20 - 30 grams on day of randomization |
| Tissue type: | NCI-H460 taxol resistant |
| Source: | Amgen Cancer Pharmacology Cell Bank |
| Subject's disease status: | Tumor Bearing Mice |
| Animal care: | AMALAR Facility |

**Test Materials**

| | |
|---|---|
| Test article: | AMG 900 |
| Manufacturer: | Amgen Inc. |
| | |
| Test article: | Taxol |
| Manufacturer: | Bristol-Myers Squibb |

**Critical Reagents Formulation**

| | |
|---|---|
| Test Compound: | AMG 900 |
| Stock Concentration: | 1.5, 0.3 mg/mL |
| Formulated: | Weekly, used within 7 days |
| Vehicle: | 2% HPMC/1% TW80 pH2.2 |
| Dose (10 mL/kg at individual bodyweight): | Dose range: 0.20 - 0.30 mL |
| Route of Administration: | PO |
| Test Compound: | Taxol |
| Stock Concentration: | 6 mg/mL |
| Formulated: | Purchased from Burt's Pharmacy |
| | Manufacturer: Bristol-Meyer's Squibb |
| Vehicle: | Saline |
| Dose (10 mL/kg at individual bodyweight): | Dose range: 0.20 - 0.30 mL |
| Route of Administration: | IP |

### Formulations

AMG 900 (free base) was formulated into suspension to concentrations of 1.5 and 0.3 mg/mL. The volume dosed was equivalent to 10 mL/kg. Taxol (Bristol Meyers Squibb) was purchased commercially from Burt's Pharmacy (Newbury Park, CA) and diluted daily from stock concentration of 6 mg/mL to 1.25 mg/mL working solution.

**Treatment Protocol**

| **Group** | **n** | **Route** | **Treatment** | **Dose (mg/kg)** | **Schedule** |
|---|---|---|---|---|---|
| 1 | 10 | PO | Vehicle | - | BID 7 days/ week x 3 wks |
| 2 | 10 | PO | AMG 900 | 3 | BID 7 days/ week x 3 wks |
| 3 | 10 | PO | AMG 900 | 15 | BID 2 days ON/5 day OFF/ week x 3 wks |
| 4 | 10 | IP | Taxol | 12.5 | QD 5 days/ week x 2 wks |

The duration of the dosing phase for AMG 900 was three weeks, and two weeks for the taxol group. The tumors were measured with a digital caliper and the mice were weighed twice per week. Tumor volumes were calculated as follows: Tumor Volume (mm³) = [(W² X L)/2] where width (W) is defined as the smaller of the 2 measurements and length (L) is defined as the larger of the 2 measurements. Tumor inhibition was calculated as follows: First; take [Initial tumor volume minus final tumor volume] for control and all treatment groups; second, take the change in treated tumor volume divided by control tumor volume, minus one and then multiply by 100. Table 5 and Figure 5 hereinbelow describe the tumor volume and tumor growth inhibition results.

**Table 5. Summary of Tumor Volume Data**

| **Group** | **Mean** | **SE** | **% Inhibition** | **Median** |
|---|---|---|---|---|
| Vehicle (HPMC) PO BID 7days | 1680 | 215 | 0.0 | 1749.7 |
| AMG 900 3mpk PO BID 7days | 771 | 104 | 60.4 | 715.6 |
| AMG 900 15mpk PO BID 2days | 689 | 107 | 65.7 | 612.5 |
| Taxol 12.5mpk IP QD 5days | 1383 | 283 | 19.7 | 1072.0 |

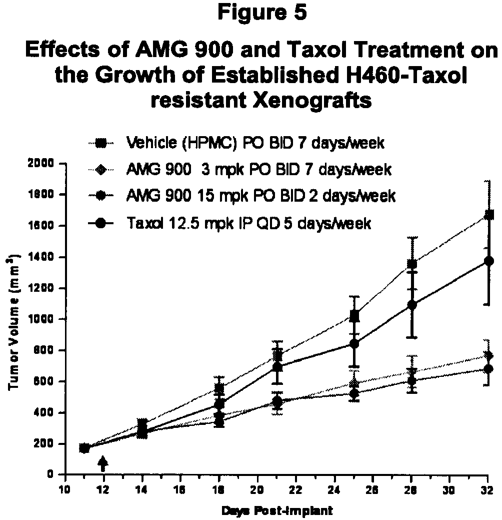

Figure 5 illustrates the effects of AMG 900 and Taxol treatment on the growth of established H460-taxol resistant tumors. Cells (2 x 10⁶ per animal) were injected subcutaneously in the right flank of female nude mice (n = 10 per group). Tumors were measured twice per week. Treatment began 12 days after tumor implantation (*arrow*) when tumors were approximately 170 mm³. Mice were dosed PO or IP once or twice daily for 3 weeks. Data represent mean ± SE for each group. *P values (not shown) correspond to statistical difference between groups treated with vehicle and AMG 900 or Taxol analyzed with repeated measure Sheffe's ANOVA over time using STATview. The scheduled days listed in the legend represent days/ week. All groups were provided nutritional supplements throughout the study.

As Figure 5 above illustrates, treatment of tumor-bearing mice with AMG 900 significantly inhibited tumor growth when administered either continuously (3 mg/kg BID for 7 days (60% inhibition, p = 0.0003)) or intermittently (15 mg/kg BID for 2 daysON/5 days OFF/week (66% inhibition, p < 0.0001)). Taxol failed to significantly inhibit tumor growth when dosed at 12.5 mg/kg IP for 5 days/week for two dosing cycles (20% inhibition, p = 0.5399) in this experiment.

It was surprising and unexpected to find that AMG 900 remains active in tumor cells lines that are resistant to three well characterized Aurora kinase inhibitors: AZD1152; VX-680 (also commonly referred to as MK-0457); and PHA-739358 (Danusertib). The inhibitors used for these experiments are compounds published and characterized in the literature. For example, see Expert Opinion Investigational Drugs (2009) 18 (4) pg 379-398; Expert Opinion Therapeutic Patents, (2009) 19 (3), pg 321-356. A detailed safety, tolerability and pK profile, including chemical structure, of Danusertib (PHA-739358) in phase I in patients with advanced or metastatic solid tumors is available in Steeghs et al, Journal of Clinical Oncology, 27, 2009.

The data presented below indicates the activity of AMG 900 in Taxol-resistant cell lines compared with the ability of the three above-mentioned, well known Aurora kinase inhibitors to inhibit phosphorylation of histone H3 in the same Taxol-resistant cells.

### Example 5

Three aurora kinase inhibitors (AZD1152, MK-0457, and PHA-739358) were evaluated in a subset of MDR tumor cell lines expressing either P-gp or BCRP drug efflux transporters. Unexpectedly, AMG 900 inhibited p-histone H3 or induced polyploidy across all the cell lines tested irrespective of P-gp or BCRP status with uniform IC₅₀ or EC₅₀ values (2 to 3 nmol/L). By contrast, the other aurora kinase inhibitors were less potent in one or more of the MDR cell lines compared with the matched sensitive tumor cell lines, as shown in Table 6 below.

### Materials and Methods

Compound Materials: Molecular structures for the following compounds: Paclitaxel and Docetaxel, MLN8054, MK-0457, AZD1152 and PHA-739358, are available in the public domain. The materials were purchased from commercial sources, where applicable, as were the Taxanes.

Cell lines: Tumor cell lines were obtained from the American Type Culture Collection (ATCC) unless otherwise specified. The MDA-MB-231-PTX and NCI-H460-PTX cell lines were established by growing the cells in the presence of increasing concentrations of paclitaxel over a period of 6 months. The HCT116 AZD1152-resistant cell line was established by growing the cells in the presence of AZD1152 at 80 nmol/L.

Animals: All experimental procedures were conducted in accordance with Institutional Animal Care and Use Committee and U.S. Department of Agriculture regulations. Four to six-week-old female athymic nude mice (Harlan Sprague Dawley) were housed in sterilized cages and maintained under aseptic conditions. The laboratory housing the animals provided alternating light and dark cycles (12 hours each) and met the standards of the Association for Assessment and Accreditation of Laboratory Animal Care specifications. Food, water, and nutritional supplements were offered *ad libitum.* All drugs were administered based on the individual body weight of each mouse.

Fluorescence-based cell imaging assays: All high-content cell assays were performed on an ArrayScan VTi HCS Reader (Cellomics). Tumor cell lines were treated with AMG 900, AZD1152, MK-0457, or PHA-739358 (concentration range varied based on potency). The cells were prepared for intracellular staining with an anti-p-histone H3 Ser¹⁰ antibody as previously described (1). Detection was performed with an anti-rabbit IgG-alexa-568 antibody and DAPI. The cellular levels of p-histone H3 were analyzed using Target Activation V2 algorithm (Cellomics) to determine the percentage of positive cells. For the imaging assays, the concentration-response curves and corresponding IC₅₀ and EC₅₀ values were calculated using the percentage of cells affected versus the DMSO control.

Colony formation assay: Tumor cells were treated with AMG 900, paclitaxel or AZD1152 (0.5, 5, 50 nmol/L) for 48 hours, washed twice with complete media, and cells were re-plated at a density of 5000 cells per well in drug-free complete media. Cells were grown until the DMSO control wells were confluent. Cells were stained with crystal violet dye (Sigma), washed with distilled water, and imaged using a digital scanner (Hewlett-Packard).

> 4N DNA content assay: Tumor cells were treated with AMG 900, AZD1152, MK-0457, or PHA-739358 (range concentration varied based on potency) for 24 hours and processed for cell-cycle analysis (DNA staining only) as previously described (1). Cells were analyzed on a LSRII flow cytometer using BD FACS Diva software. The concentration-response curves and corresponding EC₅₀ values were calculated using the percentage of cells with ≥4N DNA content versus the DMSO control.

P-gp and BCRP cell surface staining: Cell surface expression of P-gp (ABCB1) and BCRP (ABCG2) were determined by staining live cells on ice for 30 minutes with FITC-conjugated P-gp (BD Bioscience) or APC-conjugated BCRP (Millipore) antibodies. Matching isotype antibodies (BD Bioscience) were used as controls as well as the viability stain 7-aminoactinomycin D (BD Biosciences) to exclude dead cells. Cells were analyzed on a LSRII flow cytometer using BD FACS Diva software.

Aurora kinase gene analysis: Total RNA and genomic DNA were isolated from frozen HCT116 cell pellets (three AZD1152-resistant cell subclones and one parental cell control) using standard nucleic acid extraction methods. Total RNA was used to generate cDNA (Advantage RT-PCR kit, Clontech). PCR amplification of full length aurora-A and -B gene transcripts were performed using the Expand-polymerase-long-template kit (Roche). PCR primer pairs included: aurora-A (GCTTGTTACTTATTACAGCTAGAGGCATCATG and TCAAGGATTTCTCCCCCTGCACGATTC), aurora-B (TCTCCTCCCCCTTTCTCTCTAAGGATG and ACCCGAGTGAATGACAGGGACCATC). Seven exons of aurora-C were amplified using the same PCR kit as described above from genomic DNA using seven primer sets based on 5' and 3' flanking introns ((AACAGCCATCCAGAGGGTTCAGGAAG and CCACACACCCAGTCTGTTCTTCATCC), (AAGGGG AGCATTGGCATCCCTGACTTTC and GTATTTGGGGAAAATGCTGGGCTCAGAC), (ACCAGGCAGTGACGGTGGCAT CATATG and TGACAGCCACAAACAGAGCTCCCAC), (GGTAAGTGTTCCACCTCAGACGGAAATTG and CAT TAAACTGGGTCATTCCTAACTGGTACTCAG), (CTCAATGAAAGCTGGGGAAGGAGAATTTCC and AGAGGC ATTGATAGTGGAAACCTCACATC), and (ACAGTGAGACTTACAGACGCATCCTCAAG and AGGAGAGCT CCCTGAACACACACAAAG)). The PCR DNA products were subcloned into pCR2.1 vector according to the manufacturer's recommended protocol (Invitrogen). Purified plasmids containing the aurora-A, -B, and -C gene products were subjected to dideoxy cycle sequencing using flanking vector primers. Sequencing reactions were run on 3730x1 DNA Analyzers (Applied Biosystems), and the output sequences were analyzed using Sequencher software (Gene Codes Corporation).

Table 6 illustrates how AMG 900 exhibits uniform potency across multi-drug resistant tumor cell lines.

**Table 6-A**

| **Cell Line designation** | **P-gp Status** | **p-histone H3 assay, IC₅₀ value (nmol/L)** | | | |
|---|---|---|---|---|---|
| | | **AMG900** | **AZD1152** | **MK-0457** | **PHA-739358** |
| NCI-H460 parental | - | 3 | 131 | 123 | 510 |
| NCI-H460 PTX paclitaxel resistant | + | 3 | >500 | 468 | >1250 |
| MDA-MB-231 parental | - | 2 | 16 | 43 | 49 |
| MDA-MB-231 PTX paclitaxel resistant | + | 3 | > 500 | 277 | >1250 |
| MES-SA parental | - | 3 | 51 | 51 | 113 |
| MES-SA-Dx5 doxorubicin resistant | + | 2 | >500 | >1250 | >1250 |

| **Cell Line** | **BCRP Status** | **≥ 4N DNA Contentassay, EC₅₀ value (nmol/L)** | | | |
|---|---|---|---|---|---|
| | | **AMG900** | **AZD1152** | **MK-0457** | **PHA-739358** |
| U226-B1 | - | 2 | 12 | 46 | 67 |
| RPMI8226 | + | 3 | 865 | 162 | > 1250 |

| | | | | | |
|---|---|---|---|---|---|
| **Note**: The origins of NCI-H460-PTX cell line is the lung; the MDA-MB-231-PTX cell line is the breast, the MES-SA Dx5 cell line is the uterus, and the RPMI-8226 cells are from multiple myeloma. | | | | | |

Similar experiments were conducted with additional cell lines as shown in table 6-B below.

**Table 6-B**

| **Cellular EC₅₀ (nM)** | | **AMG 900** | **AZD1152-HQPA** | **PHA-739358** | **MK-0457** |
|---|---|---|---|---|---|
| **Cell line** | **Origin** | | | | |
| MCF-7 | Breast | 1 | 11 | 68 | 27 |
| NCI-H460-PTX* | Lung | 3 | >500 | >1250 | 468 |
| HCT15* | Colon | 2 | >625 | >1250 | 908 |
| MES-SA Dx5* | Uterine | 2 | >500 | >1250 | >1250 |
| MDA-MB-231-PTX* | Breast | 3 | >500 | >1250 | 277 |
| SNU499* | Liver | 2 | >1250 | >1250 | ND |
| 769P* | Kidney | 4 | 703 | >1250 | ND |
| RPMI-8226** | Multipl e Myeloma | 3 | 865 | >1250 | 162 |

| | | | | | |
|---|---|---|---|---|---|
| Note: ABC transporter status (***** = P-gp+; ****** = P-gp- and/or BCRP+) ND = not determined | | | | | |

### Example 6

In addition, AMG 900 was evaluated *in vivo* against HCT-116 cells adapted to grow in the presence of AZD1152, a selective inhibitor of Aurora kinase B. This experiment also shed some light on possible alternative mechanisms of resistance to aurora kinase inhibitors. Thus, HCT116 cells were adapted to grow in the presence of AZD1152. The activity of AMG 900 was then evaluated in the HCT116 parental and AZD1152-resistant cell lines.

AMG 900 was surprisingly found to induce polyploidy and inhibit colony formation of an HCT116 subline adapted to grow in the presence of AZD1152. The cellular ≥ 4N DNA content ECso values for AMG 900 were 2 and 5 nmol/L compared to 34 and 672 nmol/L for AZD1152, respectively (Figure 6-A). AMG 900 inhibited the colony formation in both HCT116 cell lines at concentrations ≥ 5 nmol/L, whereas the variant subline was insensitive to AZD1152 at 50 nmol/L (Figure 6-B). Both of the HCT116 cell lines were equally sensitive to paclitaxel and were negative for P-gp and BCRP expression (Figure 6-C). Interestingly, the HCT116 variant subline harbors a missense mutation in one allele of the aurora-B gene (TGG → TTG; *W221L*), whereas no mutations were detected in the aurora-A and -C genes. These results suggest that AMG 900 maintains activity in tumor cells which are resistant to AZD1152 and, more particularly, tumor cells carrying a heterozygous mutation in aurora-B that may be responsible for resistance to AZD1152. Thus, the unexpectedly positive data indicate the surprising ability of MAG 900 to remain efficacious in treating tumor cells which have become resistant to AZD1152.

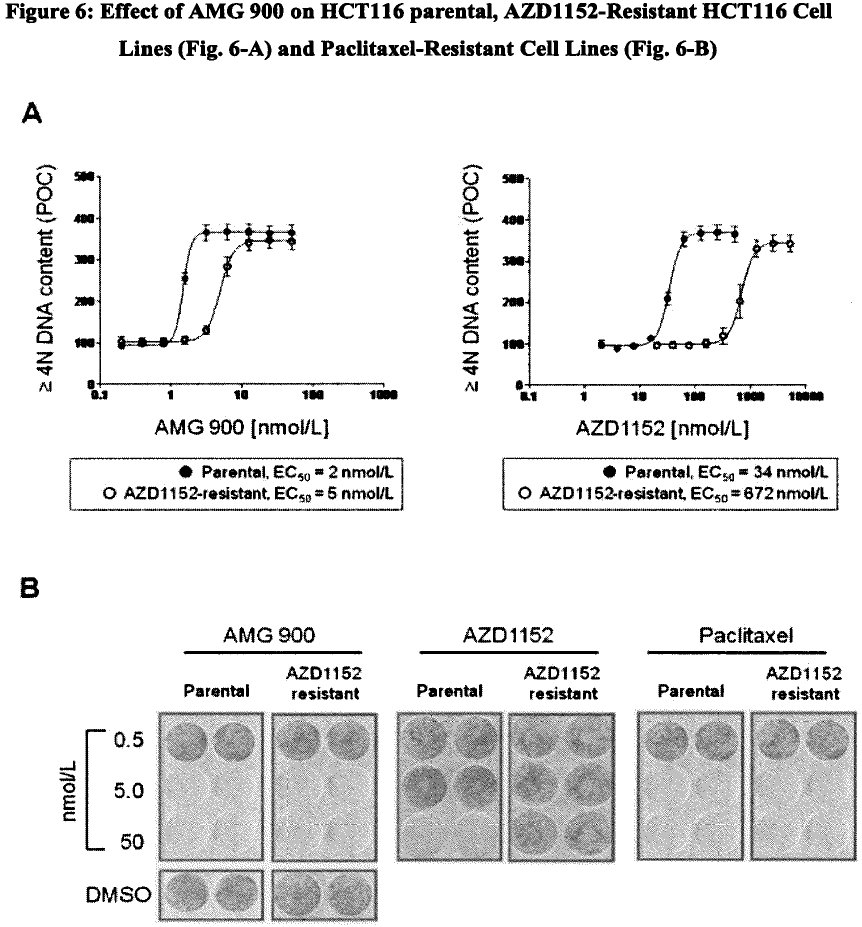

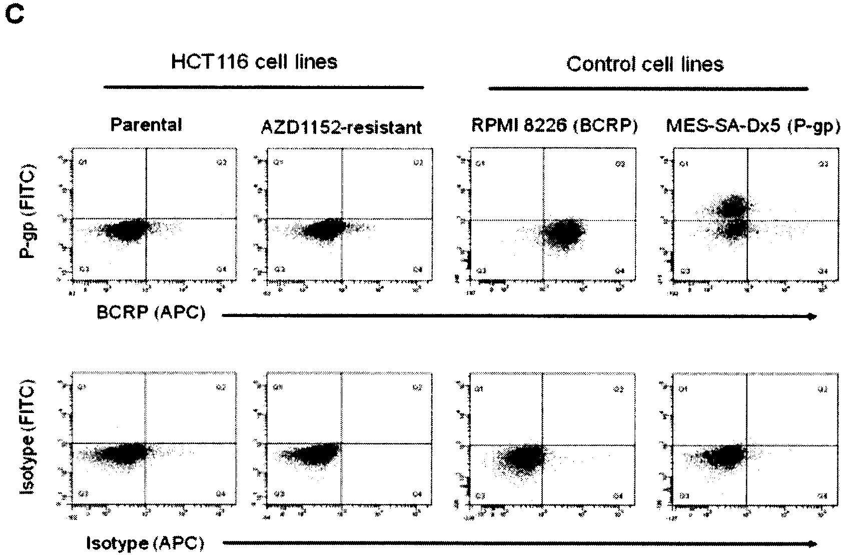

### Methods:

Fig. 6-A: HCT116 cell lines were treated with increasing concentrations of AMG 900 or AZD1152 for 24 hours. Flow cytometry was used to assess the accumulation of cells with ≥ 4N DNA content expressed as a percentage of the DMSO-treated control (POC). The concentration-response curves and calculated EC₅₀ values were determined from two independent experiments (bars, ±SD).

Fig. 6-B: Colony formation assay was performed with HCT116 cell lines (parental and AZD1152-resistant). Cells were treated with DMSO, AMG 900, AZD1152, or paclitaxel at the indicated concentrations for 48 hours and re-plated in complete media lacking the inhibitor. After the DMSO-treated cells reached confluence, the cells were stained with crystal violet and imaged (duplicate wells).

Fig. 6-C: The extent of P-gp and BCRP expression on the cell surface of HCT116 cell lines (parental and AZD1152-resistant) co-stained with phycoerythrin (PE)-conjugated P-gp and allophycocyanin (APC)-conjugated BCRP antibodies were evaluated and analyzed by flow cytometry. Isotype controls were used for each cell line to establish background fluorescence. MES-SA-Dx5 and RPMI 8226 cell lines were used as positive controls for the P-gp and BCRP expression, respectively.

### Animals:

Female athymic nude mice (Harlan Sprague Dawley) aged 5-6 weeks were received and housed in sterilized caging. Reverse osmosis water and autoclaved food were supplied ad libitum. All animal studies were performed under an internal IACUC protocol and met all AAALAC specifications.

### Pharmacodynamic Assays (Detection of phospho-Histone H3):

Mice with established human HCT 116 or Colo 205 xenograft tumors were administered a single oral dose of control (vehicle alone) or AMG 900 at the indicated dosage (n=3 animals per group). At 3 or 6 hours, tumor, bone marrow or skin tissues were collected for pharmacodynamic evaluation (p-histone H3 levels). Plasma was also collected for pharmacokinetics analysis.

Flow Cytometry (FCM): Tumors were dissociated into a single cell suspension and fixed in 90% methanol at -20°C for at least 24 hours. Cells were then stained with anti-p-histone H3 (ser-10) and anti-cytokeratin antibodies and counterstained with propidium iodide. Data was acquired on LSRII flow cytometer running FACSDiva software. Bone marrow and cytokeratin positive tumor cells in the G2M phase of the cell cycle were evaluated for p-histone H3. Tumor and bone marrow cells were collected from the vehicle-treated mice to serve as the p-histone H3 baseline controls. Statistical significance was determined by oneway ANOVA analysis.

Laser Scanning Cytometry (LSC): Triplicate sections from FFPE tissue samples (skin and tumor) were stained using anti-p-histone H3 antibody followed by an alexa-633 conjugated goat anti-rabbit IgG. Slides were mounted with Prolong Gold antifade including DNA dye Hoechst33342. Images were captured on the LSC using a 40x objective (at 0.5µ pixel resolution). The number of p-histone H3 events were determined based on a defined red fluorescent threshold. Only events larger than 20 µm2 were counted. Contoured events were relocated to an image gallery to verify the accuracy of segmentation. Data were analyzed using SAS V9.3 with Dunnett's adjustment applied. All statistical tests were evaluated at alpha = 0.05 significance level.

Fine Needle Aspirates (FNA): Tumor aspirates were collected by inserting a 25 gauge needle through a small incision in the skin surrounding the tumor in a predetermined and consistent pattern (3x), then were expelled into 2% paraformaldehyde. Cells were cytospun onto microscope slides and stained with antibodies specific for EpCAM (epithelial tumor marker, Alexa Fluor 488) and pHH3 (mitosis marker, Alexa Fluor 647) and counterstained with DAPI (DNA content). An iCyte LSC (lasers 405 nm, 488 nm, 633 nm, PMT filters: 450/40, 530/30, 650/LP) was used to capture 40x magnification field images and to quantify EpCAM, pHH3 and DNA content. The integrity of the population was verified by relocating images of cells into galleries. The numbers of EpCAM, pHH3 positive cells in G2M were reported. Data were represented as a mean +/- standard error of the mean (SEM). Statistical significance was determined using ANOVA followed by Bonferroni Dunnett's post hoc analysis.

### AMG 900 Concentration in Plasma (pharmacokinetic Assay):

Plasma samples (50 µL) were extracted by addition of a solvent mixture (90% methanol, 10% water with 0.01% trifluoroacetic acid) to isolate the analyte and precipitate plasma proteins. AMG 900 concentrations in extracted samples were determined by LC-MS/MS using reversed-phase liquid chromatography on a Varian Pursuit PFP analytical column (2.0 x 30 mm, 5 micron) with 0.1% formic acid in water (mobile phase A) and acetonitrile with 0.1% formic acid (mobile phase B).

### Xenograft Models:

Mice were injected subcutaneously with 2 x 106 human HCT 116 colon tumor cells. When tumors were established (approximately 200 mm3), mice were randomized into experimental treatment groups (n = 10) and treated orally with AMG 900 at 1.5, 2.25 or 3 mg/kg everyday or, 3.75, 7.5 or 15 mg/kg intermittently for the duration of the experiment. Mice were provided nutritional supplements on a daily basis. Tumor volumes and body weights were recorded twice per week using caliper and analytical digital scale, respectfully. Tumor data were represented by mean tumor volume +/- SEM. Statistical significance for tumor growth inhibition was determined by repeated measurements ANOVA (RMANOVA) followed by Scheffe post-hoc analysis.

### Example 7

The *in-vivo* effect of AMG 900 on tumor growth was further evaluated in a panel of human xenografts from five different tumor types (breast, colon, lung, pancreatic, and uterine), including three MDR xenograft models. Mice bearing established tumors were orally administered AMG 900 at 15 mg/kg b.i.d. for 2 consecutive days per week for 3 weeks or at 3 mg/kg b.i.d. every day for 3 weeks. The maximum percentage of tumor growth inhibition (TGI) is reported, in Table 7 below, for each xenograft model. The percentage of TGI was calculated as the difference between the change in vehicle-treated control and AMG 900-treated tumor volumes during the study period. Statistically significant tumor growth inhibition compared with the vehicle-treated control was determined by RMANOVA followed by Scheffe or Dunnett post-hoc tests and is denoted by asterisks (**P* < 0.005, ***P* < 0.0005).

AMG 900 exhibited significant antitumor activity in all 9 xenograft models (50 to 97% tumor growth inhibition (TGI) compared with the vehicle-treated control group. Importantly, AMG 900 was active in the MES-SA-Dx5 (84% TGI, *P*< 0.0001) and NCI-H460-PTX (66% TGI, *P* < 0.0001) xenograft models that were resistant to either Docetaxel or Paclitaxel administered at their respective maximum tolerated doses.

Thus, AMG 900 inhibits the growth of multiple human tumor xenografts *in vivo*, including multidrug resistant models and standard-of-care antimitotic drugs. Specifically, the data shows that AMG 900 surprisingly inhibits the activity of aurora-B in HCT116 tumors and suppresses the growth of multiple xenografts representative of diverse tumor types.

**Table 7**

| Tumor Model | Origin | TGI (%) |
|---|---|---|
| MDA-MB-231 | Breast | 82** |
| COLO 205 | Colon | 73* |
| HCT-15 (MDR) | Colon | 50* |
| HCT116 | Colon | 85** |
| NCI-H460 | Lung | 85** |
| NCI-H460-PTX (MDR) | Lung | 65** |
| MiaPaCa2 | Pancreas | 60** |
| MES-SA | Uterine | 87** |
| MES-SA-Dx5 (MDR) | Uterine | 84** |

| | | |
|---|---|---|
| **References:** Payton M, Chung G, Yakowec P, et al. Discovery and evaluation of dual CDK1 and CDK2 inhibitors. Cancer Res 2006;66:4299-4308. | | |

### INDICATIONS

The mechanisms by which tumors develop resistance to Aurora kinase inhibitors are likely to differ for different agents. While a clearer understanding the molecular forces which drive cancer phenotypes would provide the basis for molecularly targeted medicines or therapies that could exploit identifiable genetic or epigenetic susceptibilities to a given therapy, it's also crucial to understand the genetic basis for resistance to therapy. This genetic understanding may afford an additional filter with which to stratify a prospective patient population. For example, published genetic evidence suggests that the Aurora kinase inhibitor, AZD1152, which is currently undergoing clinical evaluation, and potentially another clinical compound, VX-680, may be relatively ineffective in tumor cells that over-express MDR1 or BCRP. Aurora B kinase domain mutations that emerge during selection of the DNA repair-defective colon carcinoma line, HCT116, may lead to the resistance. These catalytic domain mutations were found to be also sufficient to render cells resistant to AZD1152 and VX-680 indicating that resistance to these agents can occur independently of MDR. The Pharmacogenomics Journal, (2009) 9, pgs 90-102.

Disclosed herein is a compound, AMG 900, an Aurora kinase inhibitor, which possesses the ability to treat cancers that have become refractory to traditional, standard of care chemotherapeutic agents, including antimitotic agents, such as taxanes (paclitaxel and docetaxel) and vinca alkaloids. In addition, AMG 900 has the ability to treat cancers that are resistant to other Aurora kinase inhibiting agents, including but not limited to AZD 1152, VX-680 and PHA-739358. Generally, such tumors develop resistance as a result of previous and/or prolonged treatment with anti-cancer agents.
Accordingly, there is provided an effective dosage amount of the compound N-(4-((3-(2-amino-4-pyrimidinyl)-2-pyridinyl)oxy)phenyl)-4-(4-methyl-2-thienyl)-1-phthalazinamine for use in treating cancer in a subject, wherein the subject's cancer was previously treated with an anti-cancer agent. In another embodiment, the anti-cancer agent is a chemotherapeutic agent. In another embodiment, the chemotherapeutic agent is an antimitotic agent or an anthracycline. In yet another embodiment, the chemotherapeutic agent is an agent selected from the group consisting of taxol, docetaxel, vincristine, vinblastine, vindesine, and vinorelbine, daunorubicin, doxorubicin, idarubicin, epirubicin, and mitoxantrone. In yet another embodiment, the anti-cancer agent is AZD1152, PHA-739358, MK-0457 or a combination thereof.

As such, AMG 900 may be used to treat cellular proliferation disorders, including uncontrolled cell growth and aberrant cell cycle regulation, which also have been previously treated with taxanes standard-of-care therapies.

To this end, AMG 900 is useful for, but not limited to, the prevention or treatment of cancer including, for example, various solid and hematologically derived tumors, such as carcinomas, including, without limitation, cancer of the bladder, breast, colon, kidney, liver, lung (including small cell lung cancer), esophagus, gall-bladder, ovary, pancreas, stomach, cervix, thyroid, prostate, uterus and skin (including squamous cell carcinoma); hematopoietic tumors of lymphoid lineage (including leukemia, acute lymphocitic leukemia, acute lymphoblastic leukemia, B-cell lymphoma, T-cell-lymphoma, Hodgkin's lymphoma, non-Hodgkin's lymphoma, hairy cell lymphoma and Burkett's lymphoma); hematopoietic tumors of myeloid lineage (including acute and chronic myelogenous leukemias (AML and CML), myelodysplastic syndrome and promyelocytic leukemia); tumors of mesenchymal origin (including fibrosarcoma and rhabdomyosarcoma, and other sarcomas, e.g. soft tissue and bone); tumors of the central and peripheral nervous system (including astrocytoma, neuroblastoma, glioma and schwannomas); and other tumors (including melanoma, seminoma, teratocarcinoma, osteosarcoma, xenoderoma pigmentosum, keratoctanthoma, thyroid follicular cancer and Kaposi's sarcoma), where such cancers have relapsed or become refractory . Cancers, such as prostate cancer, ovarian cancer, lung cancer, breast cancer, cholangiocarcinoma or other types of cancer, which have become refractory to anti-cancer treatment, such as with hormones, may also be treated with AMG 900.

The cancers to be treated may be one or more selected from the group consisting of uterine cancer, breast cancer, lung cancer including non-small cell lung cancer, colon cancer, prostate cancer, skin cancer, kidney cancer, liver cancer, leukemias including promyelocytic leukemia, chronic myeloid leukemia and T-cell leukemia, multiple myeloma, ovarian cancer and bone marrow cancer in a subject, the method comprising administering to the subject an effective dosage amount of AMG 900, wherein the subject's cancer has previously been treated with and become refractory to one or more chemotherapeutic agents selected from the group consisting of doxorubicin, daunorubicin, dactinomycin, colchicine, vinblastine, vincristine, paclitaxel, docetaxel, etoposide and mitoxantrone. In another embodiment, the invention provides a method of treating one or more cancers selected from the group consisting of cancer of the bladder, breast, colon, kidney, liver, lung, non-small cell lung, head and neck, esophageal, gastric, ovarian, pancreas, stomach, cervix, thyroid and prostate or a lymphoma or leukemia. AMG 900 is also useful for treating advanced solid tumors, including without limitations, tumors of the bladder, breast, colon, kidney, liver, lung, non-small cell lung, head and neck, esophageal, gastric, ovarian, pancreas, stomach, cervix, thyroid and prostate.

Disclosed herein is a method for the treatment of solid tumors, sarcomas (especially Ewing's sarcoma and osteosarcoma), retinoblastoma, rhabdomyosarcomas, neuroblastoma, hematopoietic malignancies, including leukemia and lymphoma, tumor-induced pleural or pericardial effusions, and malignant ascites.

Besides being useful for human treatment, the compound is also useful for veterinary treatment of companion animals, exotic animals and farm animals, including mammals, rodents, and the like. For example, animals including horses, dogs, and cats may be similarly treated with AMG 900 for cancers refractory to standard-of-care cancer chemotherapy treatments.

### FORMULATIONS

AMG 900 may be administered to the cancer subject as a pharmaceutical composition, comprising the compound (which is the active pharmaceutical ingredient or API of the invention), N-(4-((3-(2-amino-4-pyrimidinyl)-2-pyridinyl)oxy)phenyl)-4-(4-methyl-2-thienyl)-1-phthalazinamine, in association with one or more non-toxic, pharmaceutically-acceptable carriers, diluents and/or adjuvants (collectively referred to herein as "excipient" materials). AMG 900, or a pharmaceutically acceptable salt form thereof, can be processed in accordance with conventional methods of pharmacy to produce the medicinal and pharmaceutical compositions for administration to patients, including humans and other mammals.

The pharmaceutical composition may be administered to the subject by any suitable route, adapted to such a route, and in a dose effective for the refractory cancer treatment intended. The composition, or API, may, for example, be administered orally, mucosally, topically, rectally, pulmonarily such as by inhalation spray, or parentally including intravascularly, intravenously, intraperitoneally, subcutaneously, intramuscularly intrasternally and infusion techniques, in dosage unit formulations containing conventional pharmaceutically acceptable carriers, adjuvants, and vehicles.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. For example, these may contain an amount of active ingredient from about 1 to 2000 mg, and typically from about 1 to 500 mg. A suitable daily dose for a human or other mammal may vary widely depending on the condition of the patient and other factors, but, once again, can be determined using routine methods and practices.

The amount of the API (AMG 900) which is administered and the dosage regimen for treating the refractory cancer condition depends on a variety of factors, including the age, weight, sex and medical condition of the subject, the type of disease, the severity of the cancer, the route and frequency of administration, and the physical and chemical properties of AMG 900 or its particular form, including the specific salt form. Thus, a dosage regimen may vary. A daily dose of about 0.01 to 500 mg/kg, advantageously between about 0.01 and about 50 mg/kg, more advantageously about 0.1 and about 30 mg/kg and even more advantageously between about 0.1 mg/kg and about 25 mg/kg body weight may be appropriate. Disclosed herein is a method of treating cancer in a subject, the method comprising administering to the subject AMG 900 or a pharmaceutically acceptable salt thereof in an effective dosage amount in the range from about 0.5 mg/kg to about 25 mg/kg, wherein the subject's cancer is refractory to treatment with an anti-mitotic agent. Further disclosed herein is a method of treating cancer in a subject, the method comprising administering to the subject AMG 900 or a pharmaceutically acceptable salt thereof in an effective dosage amount in the range from about 1.0 mg/kg to about 20 mg/kg, wherein the subject's cancer is refractory to treatment with standard of care chemotherapeutic agent, including an anti-mitotic agent. Further disclosed herein is a method of treating cancer in a subject, the method comprising administering to the subject AMG 900 or a pharmaceutically acceptable salt thereof in an effective dosage amount in the range from about 3.0 mg/kg to about 15 mg/kg, wherein the subject's cancer is refractory to treatment with an anti-mitotic agent. The daily dose can be administered in one to four doses per day.

For therapeutic purposes, AMG 900 may be combined with one or more adjuvants or "excipients" appropriate to the indicated route of administration. If administered on a per dose basis, AMG 900 may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, to form the final formulation. For example, AMG 900 and the excipient(s) may be tableted or encapsulated by known and accepted methods for convenient administration. Examples of suitable formulations include, without limitation, pills, tablets, soft and hard-shell gel capsules, troches, orally-dissolvable forms and delayed or controlled-release formulations thereof. Particularly, capsule or tablet formulations may contain one or more controlled-release agents, such as hydroxypropylmethyl cellulose, as a dispersion with the API(s).

In the case of psoriasis and other skin conditions, it may be preferable to apply a topical preparation of the AMG 900 to the affected area two to four times a day. Formulations suitable for topical administration include liquid or semi-liquid preparations suitable for penetration through the skin (*e*.*g*., liniments, lotions, ointments, creams, pastes, suspensions and the like) and drops suitable for administration to the eye, ear, or nose. A suitable topical dose of the active ingredient is 0.1 mg to 150 mg administered one to four, preferably one or two times daily. For topical administration, the API may comprise from 0.001% to 10% w/w, e.g., from 1% to 2% by weight of the formulation, although it may comprise as much as 10% w/w, but preferably not more than 5% w/w, and more preferably from 0.1% to 1% of the formulation.

When formulated in an ointment, AMG 900 may be employed with either paraffinic or a water-miscible ointment base. Alternatively, it may be formulated in a cream with an oil-in-water cream base. If desired, the aqueous phase of the cream base may include, for example at least 30% w/w of a polyhydric alcohol such as propylene glycol, butane-1,3-diol, mannitol, sorbitol, glycerol, polyethylene glycol and mixtures thereof. The topical formulation may desirably include a compound, which enhances absorption or penetration of the active ingredient through the skin or other affected areas. Examples of such dermal penetration enhancers include DMSO and related analogs.

AMG 900 can also be administered by transdermal device. Preferably transdermal administration will be accomplished using a patch either of the reservoir and porous membrane type or of a solid matrix variety. In either case, AMG 900 is delivered continuously from the reservoir or microcapsules through a membrane into the active agent permeable adhesive, which is in contact with the skin or mucosa of the recipient. If AMG 900 is absorbed through the skin, a controlled and predetermined flow of AMG 900 is administered to the recipient. In the case of microcapsules, the encapsulating agent may also function as the membrane.

The oily phase of the emulsions may be constituted from known ingredients in a known manner. While the phase may comprise merely an emulsifier, it may comprise a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make-up the so-called emulsifying wax, and the wax together with the oil and fat make up the so-called emulsifying ointment base, which forms the oily dispersed phase of the cream formulations. Emulsifiers and emulsion stabilizers suitable for use in the formulation include, for example, Tween 60, Span 80, cetostearyl alcohol, myristyl alcohol, glyceryl monostearate, sodium lauryl sulfate, glyceryl distearate alone or with a wax, or other materials well known in the art.

The choice of suitable oils or fats for the formulation is based on achieving the desired cosmetic properties, since the solubility of the API in most oils likely to be used in pharmaceutical emulsion formulations is very low. Thus, the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters may be used. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredients are dissolved or suspended in suitable carrier, especially an aqueous solvent for AMG 900. AMG 900 is preferably present in such formulations in a concentration of 0.5 to 20%, advantageously 0.5 to 10% and particularly about 1.5% w/w.

Formulations for parenteral administration may be in the form of aqueous or nonaqueous isotonic sterile injection solutions or suspensions. These solutions and suspensions may be prepared from sterile powders or granules using one or more of the carriers or diluents mentioned for use in the formulations for oral administration or by using other suitable dispersing or wetting agents and suspending agents. For example AMG 900 may be dissolved in water, polyethylene glycol, propylene glycol, ethanol, corn oil, cottonseed oil, peanut oil, sesame oil, benzyl alcohol, sodium chloride, tragacanth gum, and/or various buffers. Other adjuvants and modes of administration are well and widely known in the pharmaceutical art. AMG 900 may also be administered by injection as a composition with suitable carriers including saline, dextrose, or water, or with cyclodextrin (ie. Captisol), cosolvent solubilization (ie. propylene glycol) or micellar solubilization (ie. Tween 80).

The sterile injectible preparation may also be a sterile injectible solution or suspension in a non-toxic parenterally acceptable diluent or solvent, for example as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed, including synthetic mono- or diglycerides. In addition, fatty acids such as oleic acid find use in the preparation of injectables.

For pulmonary administration, the pharmaceutical composition may be administered in the form of an aerosol or with an inhaler including dry powder aerosol.

Suppositories for rectal administration of the drug can be prepared by mixing the drug with a suitable non-irritating excipient such as cocoa butter and polyethylene glycols that are solid at ordinary temperatures but liquid at the rectal temperature and will therefore melt in the rectum and release the drug.

The pharmaceutical compositions may be subjected to conventional pharmaceutical operations such as sterilization and/or may contain conventional adjuvants, such as preservatives, stabilizers, wetting agents, emulsifiers, buffers etc. Tablets and pills can additionally be prepared with enteric coatings. Such compositions may also comprise adjuvants, such as wetting, sweetening, flavoring, and perfuming agents.

### COMBINATIONS

While AMG 900 can be dosed or administered as the sole active pharmaceutical agent, it can also be used in combination with one or more chemotherapeutic and/or antimitotic agents. When administered as a combination, AMG 900 can be formulated as separate compositions that are administered simultaneously or sequentially at different times, or AMG 900 can be given as a single composition.

The phrase "co-therapy" (or "combination-therapy"), in defining the use of AMG 900 of the present invention and another chemotherapeutic agent, is intended to embrace administration of each agent in a sequential manner in a regimen that will provide beneficial effects of the drug combination, and is intended as well to embrace coadministration of these agents in a substantially simultaneous manner, such as in a single capsule having a fixed ratio of these active agents or in multiple, separate capsules for each agent.

Specifically, the administration of AMG 900 may be in conjunction with additional chemotherapeutic agent, including antimitotic therapies, known to those skilled in the art in the prevention or treatment of cancer. The invention is not limited in the sequence of administration, i.e, AMG 900 may be administered either prior to, simultaneous with or after administration of the known anticancer or anti-mitotic agent.
The Invention is also defined by the following embodiments of items 1-14
1. Use of the compound N-(4-((3-(2-amino-4-pyrimidinyl)-2-pyridinyl)oxy)phenyl)-4-(4-methyl-2-thienyl)-1-phthalazinamine or a pharmaceutically acceptable salt thereof, for treatment of cancer in a subject, wherein the subject's cancer is refractory to treatment with an anti-cancer agent.
2. The use of the compound of item 1 wherein the anti-cancer agent is a chemotherapeutic agent.
3. The use of the compound of item 2 wherein the chemotherapeutic agent is an agent selected from the group consisting of an antimitotic agent and an anthracycline.
4. The use of the compound of item 2 wherein the chemotherapeutic agent is an agent selected from the group consisting of taxol, docetaxel, vincristine, vinblastine, vindesine, and vinorelbine, daunorubicin, doxorubicin, idarubicin, epirubicin, and mitoxantrone.
5. The use of the compound of item 1 wherein the anti-cancer agent is AZD1152, PHA-739358, MK-0457 or a combination thereof.
6. The use according to any of items 1-5 wherein the cancer is one or more of (a) a solid or hematologically derived tumor selected from cancer of the bladder, breast, colon, kidney, liver, lung, small cell lung cancer, esophagus, gall-bladder, ovary, pancreas, stomach, cervix, thyroid, prostate and skin, (b) a hematopoietic tumor of lymphoid lineage selected from leukemia, acute lymphocitic leukemia, acute lymphoblastic leukemia, B-cell lymphoma, T-cell-lymphoma, Hodgkin's lymphoma, non-Hodgkin's lymphoma, hairy cell lymphoma and Burkett's lymphoma, (c) a hematopoietic tumor of myeloid lineage selected from acute and chronic myelogenous leukemias, myelodysplastic syndrome and promyelocytic leukemia (d) a tumor of mesenchymal origin selected from fibrosarcoma and rhabdomyosarcoma, (e) a tumor of the central and peripheral nervous system selected from astrocytoma, neuroblastoma, glioma and schwannoma, or (f) a melanoma, seminoma, teratocarcinoma, osteosarcoma, xenoderoma pigmentosum, keratoctanthoma, thyroid follicular cancer or Kaposi's sarcoma.
7. The use according to any of items 1-5 wherein the cancer is one or more of a solid tumor selected from cancer of the bladder, breast, colon, kidney, liver, lung, non-small cell lung, head and neck, esophageal, gastric, ovarian, pancreas, stomach, cervix, thyroid and prostate or a lymphoma or leukemia.
8. The use according to any of items 1-5 wherein the cancer is prostate cancer, ovarian cancer, breast cancer, cholangiocarcinoma, acute myeloid leukemia, chronic myeloid leukemia or a combination thereof.
9. Use of the compound N-(4-((3-(2-amino-4-pyrimidinyl)-2-pyridinyl)oxy)phenyl)-4-(4-methyl-2-thienyl)-1-phthalazinamine or a pharmaceutically acceptable salt thereof, for the preparation of a medicament for the treatment of cancer refractory to treatment with an anti-cancer agent.
10. Use of the compound according to item 9 for the preparation of a medicament for the treatment of cancer selected from bladder cancer, breast cancer, colon cancer, kidney cancer, liver cancer, lung cancer, non-small cell lung cancer, head and neck cancer, esophageal cancer, gastric cancer, ovarian cancer, pancreatic cancer, stomach cancer, cervical cancer, thyroid cancer, prostate cancer, lymphoma, leukemia, multiple myeloma or a combination thereof, wherein the cancer is refractory to treatment with an anti-cancer agent.
11. Use of the compound according to item 9 for reducing the size of a solid tumor in a subject, wherein the subject's tumor was previously treated with a chemotherapeutic agent selected from the group consisting of paclitaxel, docetaxcel, doxorubicin and a vinca alkaloid.
12. Use of the compound according to item 9 wherein the anti-cancer agent is a chemotherapeutic agent selected from the group consisting of an antimitotic agent and an anthracycline.
13. Use of the compound according to item 9 wherein the anti-cancer agent is a chemotherapeutic agent selected from the group consisting of taxol, docetaxel, vincristine, vinblastine, vindesine, and vinorelbine, daunorubicin, doxorubicin, idarubicin, epirubicin, and mitoxantrone.
14. Use of the compound according to item 9 wherein the anti-cancer agent is AZD1152, PHA-739358, MK-0457 or a combination thereof.

## Claims

1. The compound N-(4-((3-(2-amino-4-pyrimidinyl)-2-pyridinyl)oxy)phenyl)-4-(4-methyl-2-thienyl)-1-phthalazinamine or a pharmaceutically acceptable salt thereof for use in the treatment of cancer in a subject, wherein the cancer is selected from the group consisting of breast cancer, lung cancer, colon cancer, uterine cancer, liver cancer, kidney cancer and multiple myeloma or a combination thereof, and the cancer is refractory to prior treatment with a chemotherapeutic agent, an anti-neoplastic agent, AZD1152, PHA-739538, MK-0457 or a combination thereof.

2. Use of the compound N-(4-((3-(2-amino-4-pyrimidinyl)-2-pyridinyl)oxy)phenyl)-4-(4-methyl-2-thienyl)-1-phthalazinamine or a pharmaceutically acceptable salt thereof for the preparation of a medicament for the treatment of cancer in a subject, wherein the cancer is selected from the group consisting of breast cancer, lung cancer, colon cancer, uterine cancer, liver cancer, kidney cancer and multiple myeloma or a combination thereof, and the cancer is refractory to prior treatment with a chemotherapeutic agent, an anti-neoplastic agent, AZD1152, PHA-739538, MK-0457 or a combination thereof.

3. The use or the compound for use according to claim 1 or 2, wherein the chemotherapeutic agent is a taxanes or paclitaxel.

4. The use or the compound for use according to any one of claims 1 to 3 wherein the cancer is breast cancer, lung cancer, or multiple myeloma.

5. The use or the compound for use according to claim 1 or 2 wherein the cancer is selected from the group consisting of breast cancer, lung cancer, colon cancer, uterine cancer and multiple myeloma or a combination thereof, and the cancer is refractory to prior treatment with an anti-neoplastic agent.

6. The use or the compound for use according to claims 1, 2 or 5 wherein the anti-neoplastic agent is a doxorubicin.

7. The use or compound for use according to claim 5 wherein the cancer is uterine cancer.

8. The use or compound for use according to claim 5 wherein the prior treatment is with AZD1152, or with PHA-739538.

9. The use or compound for use according to any one of claims 1 - 8 wherein AMG 900 is administered to the patient in need of treatment in a dosage amount ranging from a daily dose of about 0,1 mg/kg to a daily dose of about 25 mg/kg of the patient.

## Patentansprüche

1. Die Verbindung N-(4-((3-(2-Amino-4-pyrimidinyl)-2-pyridinyl)oxy)phenyl)-4-(4-methyl-2-thienyl)-1-phthalazinamin oder ein pharmazeutisch verträgliches Salz davon zur Verwendung bei der Behandlung von Krebs in einem Individuum, wobei der Krebs ausgewählt ist aus der Gruppe bestehend aus Brustkrebs, Lungenkrebs, Darmkrebs, Gebärmutterkrebs, Leberkrebs, Nierenkrebs und multiples Myelom oder eine Kombination davon, und der Krebs therapieresistent für die vorhergehende Behandlung mit einem Chemotherapeutikum, einem anti-neoplastischen Mittel, AZD1152, PHA-739538, MK-0457 oder einer Kombination davon ist.

2. Verwendung der Verbindung N-(4-((3-(2-Amino-4-pyrimidinyl)-2-pyridinyl)oxy)phenyl)-4-(4-methyl-2-thienyl)-1-phthalazinamin oder ein pharmazeutisch verträgliches Salz davon zur Herstellung eines Medikaments zur Behandlung von Krebs, wobei der Krebs ausgewählt ist aus der Gruppe bestehend aus Brustkrebs, Lungenkrebs, Darmkrebs, Gebärmutterkrebs, Leberkrebs, Nierenkrebs und multiples Myelom oder eine Kombination davon, und der Krebs therapieresistent für die vorhergehende Behandlung mit einem Chemotherapeutikum, einem anti-neoplastischen Mittel, AZD1152, PHA-739538, MK-0457 oder einer Kombination davon ist.

3. Die Verwendung oder die Verbindung zur Verwendung nach Anspruch 1 oder 2, wobei das Chemotherapeutikum ein Taxan oder Paclitaxel ist.

4. Die Verwendung oder die Verbindung zur Verwendung nach einem der Ansprüche 1 bis 3, worin der Krebs Brustkrebs, Lungenkrebs oder multiples Myelom ist.

5. Die Verwendung oder die Verbindung zur Verwendung nach Anspruch 1 oder 2, wobei der Krebs ausgewählt ist aus der Gruppe bestehend aus Brustkrebs, Lungenkrebs, Darmkrebs, Gebärmutterkrebs und multiples Myelom oder einer Kombination davon, und der Krebs therapieresistent für die vorhergehende Behandlung mit einem anti-neoplastischen Mittel.

6. Die Verwendung oder die Verbindung zur Verwendung nach 1, 2 oder 5, wobei das anti-neoplastischen Mittel Doxorubicin ist.

7. Die Verwendung oder die Verbindung zur Verwendung nach Anspruch 5, wobei der Krebs Gebärmutterkrebs ist.

8. Die Verwendung oder die Verbindung zur Verwendung nach Anspruch 5, wobei die vorhergehende Behandlung mit AZD1152 oder mit PHA-739538 war.

9. Die Verwendung oder die Verbindung zur Verwendung nach einem der Ansprüche 1 - 8, wobei dem Patienten, der die Behandlung benötigt, AMG 900 in einer Dosierung im Bereich von einer täglichen Dosis von etwa 0,1 mg/kg bis zu einer täglichen Dosis von etwa 25 mg/kg des Patienten verabreicht wird.

## Revendications

1. Composé N-(4-((3-(2-amino-4-pyrimidinyl)-2-pyridinyl)oxy)phényl)-4-(4-méthyl-2-thiényl)-1-phtalazinamine ou son sel pharmaceutiquement acceptable pour l'utilisation dans le traitement du cancer chez un sujet, le cancer étant sélectionné dans le groupe constitué du cancer du sein, du cancer du poumon, du cancer du côlon, du cancer de l'utérus, du cancer du foie, du cancer du rein et du myélome multiple ou une combinaison de ceux-ci, et le cancer étant réfractaire au traitement antérieur avec un agent chimiothérapeutique, un agent anti-néoplasique, l'AZD1152, le PHA-739538, le MK-0457 ou une combinaison de ceux-ci.

2. Utilisation du composé N-(4-((3-(2-amino-4-pyrimidinyl)-2-pyridinyl)oxy)phényl)-4-(4-méthyl-2-thiényl)-1-phtalazinamine ou son sel pharmaceutiquement acceptable pour la préparation d'un médicament pour le traitement du cancer chez un sujet, le cancer étant sélectionné dans le groupe constitué du cancer du sein, du cancer du poumon, du cancer du côlon, du cancer de l'utérus, du cancer du foie, du cancer du rein et du myélome multiple ou une combinaison de ceux-ci, et le cancer étant réfractaire au traitement antérieur avec un agent chimiothérapeutique, un agent anti-néoplasique, l'AZD1152, le PHA-739538, le MK-0457 ou une combinaison de ceux-ci.

3. Utilisation ou composé pour l'utilisation selon la revendication 1 ou 2, l'agent chimiothérapeutique étant un taxane ou du paclitaxel.

4. Utilisation ou composé pour l'utilisation selon l'une quelconque des revendications 1 à 3, le cancer étant le cancer du sein, le cancer du poumon, ou le myélome multiple.

5. Utilisation ou composé pour l'utilisation selon la revendication 1 ou 2, le cancer étant sélectionné dans le groupe constitué du cancer du sein, du cancer du poumon, du cancer du côlon, du cancer de l'utérus et du myélome multiple ou une combinaison de ceux-ci, et le cancer étant réfractaire au traitement antérieur avec un agent anti-néoplasique.

6. Utilisation ou composé pour l'utilisation selon les revendications 1, 2 ou 5 dans lequel l'agent anti-néoplasique est la doxorubicine.

7. Utilisation ou composé pour l'utilisation selon la revendication 5, le cancer étant le cancer de l'utérus.

8. Utilisation ou composé pour l'utilisation selon la revendication 5, le traitement antérieur étant effectué avec de l'AZD1152, ou avec le PHA-739538.

9. Utilisation ou composé pour l'utilisation selon l'une quelconque des revendications 1 à 8, l'AMG 900 étant administré au patient ayant besoin de traitement en un niveau de dose s'étendant d'une dose quotidienne d'environ 0,1 mg/kg à une dose quotidienne d'environ 25 mg/kg du patient.


### SEQUENCE LISTING

<110> Amgen Inc.
<120> N-(4-((3-(2-Amino-4-Pyrimidinyl)-2-Pyridinyl)Oxy)Phenyl) -4-(4-Methyl-2-Thienyl)-1-Phthalazinamine for use in the Treatment of Antimitotic Agent Resistant Cancer
<130> EP81180IHV200
<140> not yet assigned
   <141> 2010-09-09
<150> PCT/US2010/048247
   <151> 2010-09-09
<150> US61/241,527
   <151> 2009-09-11
<160> 16
<170> PatentIn version 3.5
<210> 1
   <211> 32
   <212> DNA
   <213> Mus musculus
<400> 1
   gcttgttact tattacagct agaggcatca tg 32
<210> 2
   <211> 27
   <212> DNA
   <213> Mus musculus
<400> 2
   tcaaggattt ctccccctgc acgattc 27
<210> 3
   <211> 27
   <212> DNA
   <213> Mus musculus
<400> 3
   tctcctcccc ctttctctct aaggatg 27
<210> 4
   <211> 25
   <212> DNA
   <213> Mus musculus
<400> 4
   acccgagtga atgacaggga ccatc 25
<210> 5
   <211> 26
   <212> DNA
   <213> Mus musculus
<400> 5
   aacagccatc cagagggttc aggaag 26
<210> 6
   <211> 26
   <212> DNA
   <213> Mus musculus
<400> 6
   ccacacaccc agtctgttct tcatcc 26
<210> 7
   <211> 28
   <212> DNA
   <213> Mus musculus
<400> 7
   aaggggagca ttggcatccc tgactttc 28
<210> 8
   <211> 28
   <212> DNA
   <213> Mus musculus
<400> 8
   gtatttgggg aaaatgctgg gctcagac 28
<210> 9
   <211> 27
   <212> DNA
   <213> Mus musculus
<400> 9
   accaggcagt gacggtggca tcatatg 27
<210> 10
   <211> 25
   <212> DNA
   <213> Mus musculus
<400> 10
   tgacagccac aaacagagct cccac 25
<210> 11
   <211> 29
   <212> DNA
   <213> Mus musculus
<400> 11
   ggtaagtgtt ccacctcaga cggaaattg 29
<210> 12
   <211> 33
   <212> DNA
   <213> Mus musculus
<400> 12
   cattaaactg ggtcattcct aactggtact cag 33
<210> 13
   <211> 30
   <212> DNA
   <213> Mus musculus
<400> 13
   ctcaatgaaa gctggggaag gagaatttcc 30
<210> 14
   <211> 29
   <212> DNA
   <213> Mus musculus
<400> 14
   agaggcattg atagtggaaa cctcacatc 29
<210> 15
   <211> 29
   <212> DNA
   <213> Mus musculus
<400> 15
   acagtgagac ttacagacgc atcctcaag 29
<210> 16
   <211> 27
   <212> DNA
   <213> Mus musculus
<400> 16
   aggagagctc cctgaacaca cacaaag 27